# EUROPÄISCHE PATENTSCHRIFT

(11) **EP 2 734 142 B1**
(45) Veröffentlichungstag und Bekanntmachung des Hinweises auf die Patenterteilung: **28.06.2017**
(21) Anmeldenummer: 12737549.1
(22) Anmeldetag: 19.07.2012
(51) Int. Cl.: A61C 1/08

(54) **BELEUCHTUNGSVORRICHTUNG FÜR EIN MEDIZINISCHES, INSBESONDERE DENTALES, INSTRUMENT**
ILLUMINATING DEVICE FOR A MEDICAL, IN PARTICULAR DENTAL INSTRUMENT
DISPOSITIF D'ÉCLAIRAGE POUR INSTRUMENT MÉDICAL, NOTAMMENT DENTAIRE

(30) Priorität: 19.07.2011 EP 11174505
(43) Veröffentlichungstag der Anmeldung: 28.05.2014
(73) Patentinhaber: W & H Dentalwerk Bürmoos GmbH, 5111 Bürmoos (AT)
(72) Erfinder: HEINRICH, Christoph, 5110 Oberndorf (AT); JINDRA, Thomas, 5121 Ostermiething (AT)
(74) Vertreter: Benda, Ralf
(86) Internationale Anmeldenummer: PCT/EP2012/064127
(87) Internationale Veröffentlichungsnummer: WO 2013/011075

(56) Entgegenhaltungen:
- EP-A1- 0 914 809
- EP-A1- 2 420 198
- WO-A1-2010/078368
- DE-A1-102008 008 535
- DE-U1-202005 020 763
- JP-A- 2006 004 987
- US-A- 3 109 238

## Beschreibung

Die vorliegende Erfindung betrifft eine Beleuchtungsvorrichtung für ein medizinisches, insbesondere dentales, Instrument, insbesondere für einen Instrumentenkopf eines derartigen Instruments, mit zumindest einem Halbleiterelement, das zur Emission von elektromagnetischer Strahlung, insbesondere von Strahlung im sichtbaren Wellenlängenbereich, ausgebildet ist.

Eine Beleuchtungsvorrichtung für ein medizinisches, insbesondere dentales, Instrument ist zum Beispiel aus dem Gebrauchsmuster DE 20 202 020 763 U1 bekannt. Die Beleuchtungsvorrichtung umfasst eine metallische Kappe mit einem Lichtabgabefenster und einen mit der metallischen Kappe verschweißten metallischen Sockel, so dass durch die Kappe und den Sockel ein gekapselter Innenraum gebildet wird. In diesem Innenraum ist ein auf dem Sockel befestigtes, optisches Halbleiterelement (LED) zur Erzeugung von Licht vorgesehen. Zwei metallische, elektrische Kontakte durchdringen den Sockel und sind mit dem optischen Halbleiterelement zwecks Energieversorgung verbunden. Die beiden Kontakte sind im Bereich des Sockels glasversiegelt. Ein derartiger Aufbau der Beleuchtungsvorrichtung hat sich in der Praxis bewährt und schützt das in dem gekapselten Innenraum aufgenommene optische Halbleiterelement hervorragend vor aggressiven Umweltbedingungen, zum Beispiel bei der Reinigung der Instruments in einem Sterilisator, und vor Verschmutzungen. Die Kapselung bedingt jedoch eine Vergrößerung der Beleuchtungsvorrichtung und somit des Raumbedarfs der Beleuchtungsvorrichtung im oder am Instrument.

Es besteht somit die Aufgabe, eine Beleuchtungsvorrichtung mit verringerten Außenabmessungen unter Beibehaltung des bewährten, den Sockel, die Kappe, das Lichtabgabefenster und den gekapselten Innenraum umfassenden Aufbaus zu schaffen, um die Anordnung einer oder mehrerer derartiger Beleuchtungsvorrichtung in oder an dem medizinischen, insbesondere dentalen, Instrument oder an bestimmten Abschnitten oder Bauteilen eines Instruments zu erleichtern oder zu ermöglichen.

Diese Aufgabe wird gemäß einem Ausführungsbeispiel durch ein medizinisches, insbesondere dentales, Instrument, vorzugsweise durch ein medizinisches, insbesondere dentales, Handstück, mit einer Beleuchtungsvorrichtung und durch ein Verfahren zur Herstellung eines entsprechenden medizinischen, insbesondere dentalen, Instruments, vorzugsweise Handstücks, gelöst, wobei die Beleuchtungsvorrichtung umfassen kann:
Eine hohle, metallische Hülse mit einer Durchbohrung, ein transparentes Fenster zur Abgabe elektromagnetischer Strahlung, insbesondere von sichtbarem Licht, und einen Sockel, auf dem zumindest ein optisches Halbleiterelement angeordnet ist, welches ausgebildet ist, elektromagnetische Strahlung, insbesondere sichtbares Licht, zu emittieren, wobei das transparente Fenster und der Sockel die Durchbohrung der hohlen, metallischen Hülse derart verschließen, dass eine Kammer gebildet ist, in welcher das zumindest eine optische Halbleiterelement aufgenommen ist, wobei der Sockel aus keramischem Material oder aus glaskeramischem Material oder aus Glas hergestellt ist. Vorzugsweise ist ein Metall und Glas aufweisendes Material und / oder ein Verguß- oder Klebematerial, zum Beispiel ein Silikon- oder Epoxidharz oder ein Silikon- oder Epoxidkleber, zur Verbindung des Sockels mit der hohlen, metallischen Hülse vorgesehen. Das Metall und Glas aufweisende Material ist entweder zur direkten oder zur indirekten Verbindung des Sockels mit der Hülse vorgesehen. Bei der indirekten Verbindung ist das Metall und Glas aufweisende Material insbesondere als Träger(material) oder Basis(material) oder als Haftvermittler für ein weiteres Material, insbesondere für eine Metalllegierung oder eine Metalllot, ausgebildet.

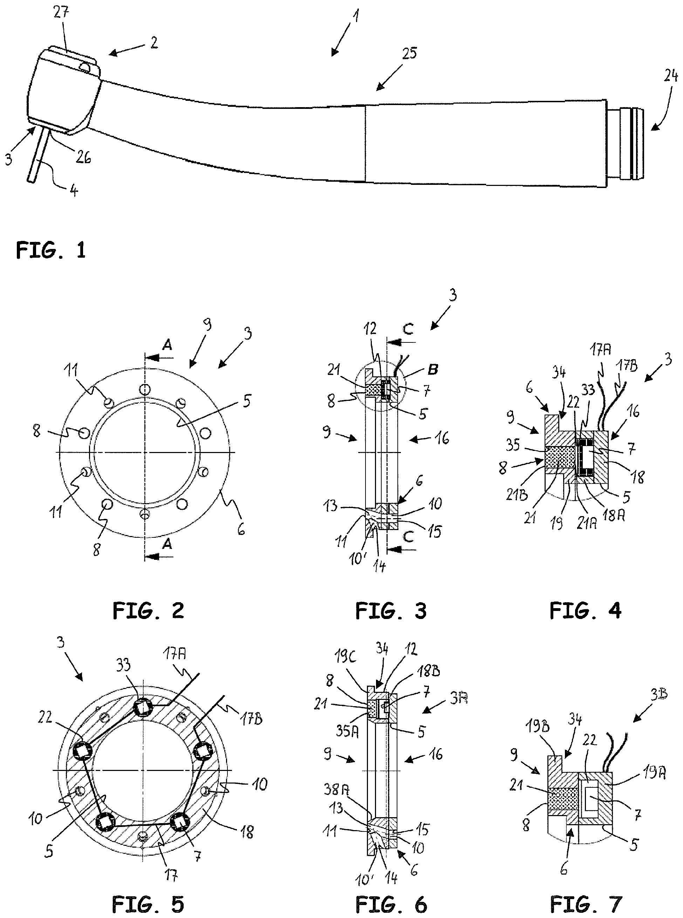

Die Beleuchtungsvorrichtung kann unter Beibehaltung des bekannten und sowohl in der Anwendung als auch in der Herstellung bewährten Aufbaus aus diskreten oder einzelnen, miteinander verbundenen Bauteilen bestehen, insbesondere aus einer hohlen, metallischen Hülse und einem Sockel. Erfindungsgemäß und von der aus dem Stand der Technik bekannten Lehre abweichend ist der Sockel aus keramischem Material oder aus glaskeramischem Material oder aus Glas hergestellt, wodurch eine erste Verkleinerung des Außendurchmessers oder Außenquerschnitts der Beleuchtungsvorrichtung erreicht wird: In dem im Vorstehenden beschriebenen Dokument DE 20 202 020 763 U1 (= DE'763) ist offenbart, die Hülse und den Sockel aus Metall zu fertigen und miteinander zu verschweißen, wozu an der Hülse ein in der Figur 1 der DE'763 mit dem Zeichen 82 versehener Flansch und an dem Sockel ein Gegenflansch vorgesehen sind. Der Flansch und Gegenflansch werden zum Schweißen, insbesondere zum Widerstandsschweißen, unter anderem benötigt, um die Schweißelektroden ansetzen zu können. Wie aus der Figur 1 der DE'763 gut zu erkennen ist, bestimmen der Flansch und Gegenflansch den größten Außendurchmesser der Beleuchtungsvorrichtung.

Durch die Verwendung eines Sockels aus keramischem Material oder aus glaskeramischem Material oder aus Glas, einer hohlen, metallischen Hülse und insbesondere durch die Verwendung eines Metall und Glas aufweisenden Materials und / oder ein Verguß- oder Klebematerials zum Verbinden des Sockels und der Hülse entfällt die Notwendigkeit, den Sockel und die Hülse mit einem Flansch und einem Gegenflansch zu versehen, wodurch der Außendurchmesser oder Außenquerschnitt der Beleuchtungsvorrichtung verringert wird. Die Wandstärke der Hülsenwand der hohlen, metallischen Hülse ist ausreichend, um den Sockel und die Hülse miteinander zu verbinden, insbesondere dicht miteinander zu verbinden, um eine abgedichtete Kammer für das zumindest eine optische Halbleiterelement bilden zu können. Der Sockel kann eine runde oder eckige, zum Beispiel vier-, sechs- oder achteckige, Außenform aufweisen.

Eine weitere Verkleinerung des Außendurchmessers oder Außenquerschnitts der Beleuchtungsvorrichtung wird durch ein bevorzugtes Ausführungsbeispiel erzielt, bei dem zumindest ein elektrischer Kontakt, der zur Verbindung des optischen Halbleiterelements mit einer elektrischen Energiequelle ausgebildet ist und der den Sockel durchsetzt, ein Metall und Glas aufweisendes Material, insbesondere ein Gemisch aus Metall- und Glaspartikeln, umfasst. Aus dem Dokument DE'763 ist bekannt, als elektrische Kontakte metallische Stifte zu verwenden, die durch Bohrungen im Sockel geführt sind und zur Befestigung, Abdichtung und elektrischen Isolierung mit einer Glasversiegelung versehen sind (siehe Absatz 61 des Dokuments DE'763). Durch die Verwendung eines Sockels aus keramischem Material oder aus glaskeramischem Material oder aus Glas und eines ein Metall und Glas aufweisenden Materials für den den Sockel durchsetzenden elektrischen Kontakt kann die Glasversiegelung entfallen, wodurch der Außendurchmesser oder Außenquerschnitt der Beleuchtungsvorrichtung zusätzlich reduziert wird.

Gemäß einem weiteren Ausführungsbeispiel umfasst die Beleuchtungsvorrichtung zumindest einen elektrischen Flächenkontakt zur Verbindung des optischen Halbleiterelements mit einer elektrischen Energiequelle auf zumindest einer Oberfläche des Sockels, wobei der elektrische Flächenkontakt ebenfalls ein Metall und Glas aufweisendes Material umfasst.

Gemäß einem Ausführungsbeispiel ist das Metall und Glas aufweisende Material, insbesondere vor einer Erwärmungs- oder Sinterbehandlung, als pastöses Material ausgebildet. Gemäß einem anderen Ausführungsbeispiel ist das Metall und Glas aufweisende, insbesondere pastöse, Material auf einer Oberfläche und / oder einem Rücksprung und / oder einer Bohrung und / oder einer Aussparung des Sockels und / oder der hohlen, metallischen Hülse aufbringbar oder aufgebracht, insbesondere aufstreichbar. Bevorzugt wird das Metall und Glas aufweisende Material vor einer Erwärmungs- oder Sinterbehandlung des Sockels auf den Sockel aufgetragen. Bevorzugt ist das Metall und Glas aufweisende Material durch Erwärmen oder Sintern mit dem Sockel verbindbar oder verbunden. Besonders bevorzugt ist durch das Erwärmen, insbesondere durch das Sintern, des Metall und Glas aufweisenden Materials, insbesondere des Glases, eine dichte und / oder feste und / oder haftende Verbindung mit dem Sockel herstellbar oder gebildet. Gemäß einem Ausführungsbeispiel beträgt die Temperatur während der Erwärmungs- oder Sinterbehandlung des Sockels in etwa zwischen 800°C - 1.900°C. Gemäß einem bevorzugten Ausführungsbeispiel ist die Schmelztemperatur des Glases des Metall und Glas aufweisenden Materials geringer als die Temperatur der Erwärmungs- oder Sinterbehandlung des Sockels, so dass das Glas zumindest teilweise schmilzt und / oder sich zumindest teilweise mit dem Sockel verbindet (anbäckt), wodurch zum Beispiel die dichte und / oder feste und / oder haftende Verbindung des Metall und Glas aufweisenden Materials mit dem Sockel entsteht.

Gemäß einem Ausführungsbeispiel ist das Metall und Glas aufweisende Material als, insbesondere pastöses, Gemisch von Metall und Glas ausgebildet, insbesondere als Gemisch von Metallpulver oder Metallpartikeln und Glaspulver oder Glaspartikeln. Vorzugsweise enthält das Gemisch des Weiteren ein Lösungsmittel, zum Beispiel Alkohol. Gemäß einem Ausführungsbeispiel umfasst das Metall und Glas aufweisende Material Metall, insbesondere Metallpulver oder körnige Metallpartikel, mit einem Anteil von zumindest etwa 50 Gew.%. Gemäß einem anderen Ausführungsbeispiel umfasst das Metall und Glas aufweisende Material Glas, insbesondere Glaspulver oder körnige Glaspartikel, mit einem Anteil von zumindest etwa 5 Gew.%. Gemäß einem Ausführungsbeispiel umfasst das Metall oder Metallpulver des Metall und Glas aufweisenden Materials zumindest eines der folgenden Metalle: Gold, Silber, Kupfer, Platin, Palladium, Wolfram, Nickel oder Molybdän. Gemäß einem Ausführungsbeispiel umfasst das Glas oder Glaspulver des Metall und Glas aufweisenden Materials zum Beispiel ein Oxidglas oder ein Silikatglas, zum Beispiel Siliziumoxid oder Borsilikat.

Wie im Vorstehenden bereits beschrieben ist das Metall und Glas aufweisende Material vorzugsweise auf dem Sockel und / oder in zumindest einer Bohrung des Sockels aufgetragen. Das in zumindest einer Bohrung des Sockels aufgenommene Metall und Glas aufweisende Material bildet insbesondere eine elektrische Verbindung oder Kontaktierung durch den Sockel zur Versorgung des zumindest einen optischen Halbleiterelements. Bevorzugt ist / wird jenes Ende oder jener Abschnitt der hohlen, metallischen Hülse, das / der mit dem Sockel verbunden ist / wird, auch mit dem Metall und Glas aufweisende Material versehen. Das Auftragen des Metall und Glas aufweisenden Materials erfolgt zum Beispiel durch Siebdruck, vorzugsweise unter Erzeugung eines Unterdrucks.

Vorzugsweise weisen zumindest einiger der im Vorstehenden genannten Ausführungsbeispiele das gleiche Metall und Glas aufweisende Material auf, insbesondere ist es zumindest bei einigen der im Vorstehenden genannten Ausführungsbeispiele als pastöses Material ausgebildet und / oder es weist die gleichen Eigenschaften und / oder Zusammensetzungen auf.

Gemäß einem Ausführungsbeispiel ist der Sockel aus keramischem Material gebildet, wobei das keramische Material zum Beispiel Aluminiumoxid oder Aluminiumnitrid aufweist. Selbstverständlich kann der Sockel auch andere keramische Materialien umfassen. Gemäß einem Ausführungsbeispiel ist der Sockel aus Glas gebildet, wobei das Glas zum Beispiel ein Oxidglas oder ein Silikatglas aufweist, zum Beispiel Siliziumoxid oder Borsilikat. Selbstverständlich kann der Sockel auch andere Glasmaterialien umfassen. Gemäß einem Ausführungsbeispiel umfasst die hohle, metallische Hülse eine Eisen-Nickel-Kobalt Legierung. Selbstverständlich kann die hohle, metallische Hülse auch andere Metalle oder Metalllegierungen umfassen. Gemäß einem Ausführungsbeispiel umfasst das transparente Fenster ein Oxidglas oder ein Silikatglas oder ein Chalkogenidglas, zum Beispiel Siliziumoxid oder Borsilikat. Vorzugsweise umfasst das transparente Fenster des Weiteren ein optisches Element, zum Beispiel eine Linse oder einen Strahlungsleiter, oder ist mit eine derartigen optischen Element verbunden. Bevorzugt ist das transparente Fenster in die hohle, metallische Hülse eingeschmolzen oder durch ein Glaslot mit der Hülse verbunden.

Gemäß einem Ausführungsbeispiel ist in der durch die hohle, metallische Hülse, den Sockel und das transparente Fenster gebildeten Kammer neben dem optischen Halbleiterelement zumindest noch ein weiteres optisches Bauteil vorgesehen, zum Beispiel ein Reflektor oder ein Konversionsmaterial zur Umwandlung der von dem optischen Halbleiterelement emittierten elektromagnetischen Strahlung, insbesondere zur Umwandlung der Wellenlänge.

Gemäß einem Ausführungsbeispiel ist der Außendurchmesser der Beleuchtungsvorrichtung kleiner als etwa 2,5 mm, bevorzugt in etwa 2,3 - 2,1 mm. Gemäß einem anderen Ausführungsbeispiel ist die Höhe der Beleuchtungsvorrichtung kleiner als etwa 2,0 mm, bevorzugt etwa 1,8 - 1,6 mm.

Gemäß einem Ausführungsbeispiel sind der Sockel, insbesondere das darauf aufgebrachte Metall und Glas aufweisende Material, und die hohle, metallische Hülse, insbesondere das darauf aufgebrachte Metall und Glas aufweisende Material, durch eine Metalllegierung miteinander verbunden, insbesondere durch ein Metalllot miteinander verlötet. Die Metalllegierung umfasst zum Beispiel zumindest eines der folgenden Metalle: Blei, Zinn, Zink, Silber und Kupfer. Vorzugsweise sind die Metalllegierung oder das Metalllot vor dem Löten pastös oder als diskretes, festes Bauteil ausgebildet. Nach dem Aufbringen der Metalllegierung oder des Metalllots zumindest auf dem Sockel und dem Anordnen der hohlen, metallischen Hülse und dem Sockel, wird die Metalllegierung oder das Metalllot über ihren Schmelzpunkt erwärmt, wodurch nach der Abkühlung eine dichte und / oder haftende und / oder feste Verbindung zwischen der hohlen, metallischen Hülse auf dem Sockel gebildet ist / wird. Gemäß diesem Ausführungsbeispiel umfasst die Verbindung zwischen der Hülse und dem Sockel somit zwei unterschiedliche Materialien/-gemische oder Werkstoffe/Werkstoffgemische, die insbesondere unterschiedliche oder aufeinander angeordnete Schichten aufweisen oder schichtweise angeordnet sind, nämlich das Metall und Glas aufweisende Material und die Metalllegierung.

Gemäß einem Ausführungsbeispiel umfasst der zumindest eine elektrische Flächenkontakt mehrere Schichten, wobei insbesondere eine erste Schicht das Metall und Glas aufweisende Material und eine zweite auf der ersten Schicht aufgebrachte Schicht ein Metall aufweist. Vorzugsweise ist / wird das Metall und Glas aufweisende Material direkt auf dem Sockel aufgebracht, insbesondere vor dem Erwärmen oder Sintern des Sockels, und die zweite Schicht ist / wird auf der ersten Schicht aufgebracht, insbesondere in einem weiteren Arbeitsgang nach dem Erwärmen oder Sintern des Sockels. Die zweite Schicht umfasst insbesondere Gold oder eine goldhaltige Legierung.

Gemäß einem Ausführungsbeispiel ist auf der zumindest einen Oberfläche des Sockels mit dem zumindest einen elektrischen Flächenkontakt das zumindest eine optische Halbleiterelement angeordnet. Gemäß einem anderen Ausführungsbeispiel bildet die zumindest eine Oberfläche des Sockels mit dem zumindest einen elektrischen Flächenkontakt eine Außenseite der Beleuchtungsvorrichtung.

Ein Verfahren zur Herstellung eines medizinischen, insbesondere dentalen, Instruments, vorzugsweise eines medizinischen, insbesondere dentalen, Handstücks, mit einer Beleuchtungsvorrichtung, umfasst die Schritte:
- Zur Verfügung stellen eines medizinischen, insbesondere dentalen, Instruments, vorzugsweise eines medizinischen, insbesondere dentalen, Handstücks,
- Zur Verfügung stellen einer Beleuchtungsvorrichtung, die umfasst: Eine hohle, metallische Hülse mit einer Durchbohrung, ein transparentes Fenster zum Abstrahlen elektromagnetischer Strahlung, insbesondere von sichtbarem Licht, einen Sockel aus keramischem Material oder aus glaskeramischem Material oder aus Glas, auf dem zumindest ein optisches Halbleiterelement angeordnet ist, welches ausgebildet ist, elektromagnetische Strahlung, insbesondere sichtbares Licht, zu emittieren, wobei das transparente Fenster und der Sockel die Durchbohrung der hohlen, metallischen Hülse derart verschließen, dass eine Kammer gebildet ist, in welcher das zumindest eine optische Halbleiterelement aufgenommen ist,
- Befestigen der Beleuchtungsvorrichtung an oder in dem medizinischen, insbesondere dentalen, Instrument, vorzugsweise dem medizinischen, insbesondere dentalen, Handstück.

Vorzugsweise werden der Sockel mit der hohlen, metallischen Hülse durch ein Metall und Glas aufweisendes Material und / oder durch ein Verguß- oder Klebematerial, zum Beispiel ein Silikon- oder Epoxidharz oder ein Silikon- oder Epoxidkleber, verbunden. Das Verfahren umfasst insbesondere zumindest einen weiteren der folgenden Schritte, wobei in Bezug auf ein Herstellungsverfahren für die Beleuchtungsvorrichtung die Schritte bevorzugt in der im Folgenden beschriebenen Reihenfolge erfolgen:
- Metall und Glas aufweisendes Material wird auf dem Sockel aufgebracht und / oder in einer Bohrung des Sockels eingebracht, um zumindest einen elektrischen Kontakt zur Verbindung des optischen Halbleiterelements mit einer elektrischen Energiequelle zu bilden oder herzustellen, insbesondere zumindest einen elektrischen Kontakt, der den Sockel durchsetzt und / oder zumindest einen elektrischen Flächenkontakt auf zumindest einer Oberfläche des Sockels.
- durch Erwärmen, insbesondere durch Sintern, wird eine haftende und / oder feste und / oder dichte Verbindung zwischen dem Sockel und dem Metall und Glas aufweisenden Material hergestellt. Umfasst der Sockel keramisches Material, so wird durch das Erwärmen oder Sintern vorzugsweise auch der Sockel umgeformt, insbesondere erhält der Sockel seine finale, für den Einbau in die Beleuchtungsvorrichtung geeignete Form und / oder Größe.
- es wird auf eine erste Schicht, die das Metall und Glas aufweisende Material aufweist, eine zweite Schicht, die Metall aufweist, aufgebracht, so dass der zumindest eine Metall und Glas aufweisendes Material umfassende elektrische Kontakt zur Verbindung des optischen Halbleiterelements mit einer elektrischen Energiequelle, insbesondere der Flächenkontakt, mehrere Schichten umfasst.
- der Sockel, insbesondere das darauf aufgebrachte Metall und Glas aufweisende Material, und die hohle, metallische Hülse, insbesondere das darauf aufgebrachte Metall und Glas aufweisende Material, werden durch eine Metalllegierung miteinander verbunden, insbesondere durch ein Metalllot miteinander verlötet.

US 3,109,238 A1 offenbart eine Beleuchtungsvorrichtung an einem Instrumentenkopf eines dentalmedizinischen Instruments, wobei an dem Instrumentenkopf ein Werkzeug zum Einwirken auf eine Behandlungsstelle vorgesehen ist und die Beleuchtungsvorrichtung umfasst: Eine Aufnahme oder Öffnung, in welcher das Werkzeug aufnehmbar ist, einen die Aufnahme oder Öffnung umgebenden Körper, welcher ein Strahlungsabgabeende mit einer optisch leitenden, transparenten Strahlungsabgabefläche, ein Instrumentenanschlussende und eine sich zwischen dem Strahlungsabgabeende und dem Instrumentenanschlussende erstreckende Mantelfläche aufweist, ein am Körper vorgesehenes Beleuchtungselement zur Emission von elektromagnetischer Strahlung, so dass elektromagnetische Strahlung von der optisch leitenden Strahlungsabgabefläche des Strahlungsabgabeendes abgebbar ist und eine am Strahlungsabgabeende des Körpers der Beleuchtungsvorrichtung vorgesehene Öffnung zur Abgabe von Fluid, so dass vom Strahlungsabgabeende des Körpers zusätzlich ein Fluid abgebbar ist.

Ein Instrument in Form eines dentalen Handstücks mit einer Beleuchtungsvorrichtung ist zum Beispiel aus der Patentanmeldung EP 1 093 765 A2 bekannt. Gemäß einem Ausführungsbeispiel weist das Handstück einen Kopf auf, an dessen Unterseite eine Öffnung zur Abgabe eines Fluids, insbesondere von Luft oder Wasser, vorgesehen ist. Am Kopf des Handstücks, um eine Öffnung zur Aufnahme eines Behandlungswerkzeugs, ist des Weiteren eine Beleuchtungsvorrichtung mit Halbleiterelementen (LEDs) vorgesehen. Die Beleuchtungsvorrichtung mit den Halbleiterelementen ist hufeisenförmig oder C-förmig geformt und weist somit zwei freie Enden und einen Freiraum zwischen den beiden freien Enden auf. Dieser Freiraum dient zur Aufnahme der an der Unterseite des Handstückkopfs angeordneten Fluidabgabeöffnung sowie als Expansionsvolumen des sich von der Fluidabgabeöffnung kegelförmigen ausbreitenden Fluidstroms.

Durch den Freiraum zwischen den beiden freien Enden der Beleuchtungsvorrichtung ist in nachteiliger Weise ein Abschnitt am Handstück geschaffen, von dem keine Strahlung auf die Behandlungsstelle abgegeben wird, wodurch auf der Behandlungsstelle ein geringer oder schlechter ausgeleuchteter Bereich entsteht. Dies ist insbesondere nachteilig, da durch die C-förmige Ausbildung der Beleuchtungsvorrichtung auf der Behandlungsstelle eine im Wesentlichen gleichmäßige Beleuchtung geschaffen ist, an die sich das Auge des Anwenders anpasst.

Eine weitere Aufgabe besteht somit darin, eine Beleuchtungsvorrichtung mit zumindest einem Halbleiterelement für ein medizinisches, insbesondere dentales, Instrument, insbesondere für einen Instrumentenkopf eines derartigen Instruments, oder ein medizinisches, insbesondere dentales, Instrument oder einen medizinischen, insbesondere dentalen, Instrumentenkopf mit einer derartigen Beleuchtungsvorrichtung unter Vermeidung der oben genannten Nachteile zu schaffen. Die Beleuchtungsvorrichtung soll insbesondere um ein mit dem Instrument oder dem Instrumentenkopf verbindbares, auf eine Behandlungsstelle einwirkendes Werkzeug oder um eine Aufnahmeöffnung für ein derartiges Werkzeug am Instrument oder Instrumentenkopf anordenbar sein, um eine wirkungsvolle Bestrahlung oder Beleuchtung der Behandlungsstelle zu erreichen, und des Weiteren eine effektive Versorgung der Behandlungsstelle mit einem oder mehreren Behandlungsfluiden zulassen.

Diese Aufgabe wird durch eine Beleuchtungsvorrichtung nach Anspruch 1 gelöst. Durch das Vorsehen zumindest eines Fluidkanals oder zumindest einer Öffnung zur Abgabe von Fluid an der Beleuchtungsvorrichtung wird in vorteilhafter Weise eine gleichmäßige Strahlungs- und Fluidabgabe von dem Instrument oder dem Instrumentenkopf und insbesondere eine gleichmäßige und effektive Bestrahlung und eine gleichmäßige und effektive Fluidbeaufschlagung der Behandlungsstelle erzielt. Damit wird in vorteilhafter Weise eine gleich bleibende Ausleuchtung der Behandlungsstelle, an die sich insbesondere das Auge des Anwenders gut anpassen kann, und eine zuverlässige Kühlung der Behandlungsstelle und gegebenenfalls auch des Werkzeugs ermöglicht.

Der Begriff Instrument umfasst insbesondere mit einer Hand greifbare medizinische oder dentale Geräte, zum Beispiel gerade, pistolenförmige, gewinkelte oder gebogene Handgriffe, die im Dentalbereich oftmals als Winkelstücke bezeichnet werden, Teile von Handstücken oder Handgriffen, insbesondere einen Kopfabschnitt, der zum Beispiel mit einem davon lösbaren Griffabschnitt verbindbar ist, sowie Kupplungen, Adapter, Verbindungsstücke und Antriebseinheiten, zum Beispiel elektrische oder pneumatische Motoren. Unter der Bezeichnung Instrument werden des Weiteren sowohl schnurlose Instrumente verstanden, insbesondere mit einer austauschbaren oder aufladbaren Energiequelle, als auch Instrumente, die eine Versorgungsleitung und eine damit verbundene Steuer-, Regel- und / oder Versorgungseinheit umfassen.

Der Begriff Werkzeug umfasst insbesondere alle auf eine Behandlungsstelle einwirkenden Vorrichtungen, zum Beispiel Bohrer, Sägen, Zahnsteinentferner, Reamer, Feilen etc., aber auch elektromagnetische Strahlung, die auf eine Behandlungsstelle einwirkt, zum Beispiel Laserstrahlung, ein Partikelstrahl, zum Beispiel ein abrasive Partikel aufweisender Partikelstrahl, oder ein Fluidstrahl, zum Beispiel ein Wasserstrahl.

Das zur Emission von elektromagnetischer Strahlung ausgebildete Halbleiterelement ist vorzugsweise als Leuchtdiode (LED) ausgebildet. Die von dem Halbleiterelement emittierte Strahlung umfasst insbesondere Wellenlängen im Bereich zwischen etwa 380 nm - 780 nm, also im Wesentlichen sichtbares Licht. Die Beleuchtungsvorrichtung wird somit vorzugsweise für die Beleuchtung einer mit dem Instrument oder Instrumentenkopf behandelten Behandlungsstelle mit sichtbarem Licht verwendet. Alternativ ist das Halbleiterelement ausgebildet, Strahlung zum Aushärten lichthärtbarer Materialien abzugeben, bevorzugt Wellenlängen von in etwa 440 nm - 480 nm, oder Strahlung zum Nachweis von Karies oder Plaque zu emittieren, vorzugsweise Wellenlängen von in etwa 390 nm - 420 nm. Bevorzugt umfasst die Beleuchtungsvorrichtung mehrere Halbleiterelemente, die Strahlung mit einem ersten und einem zweiten, unterschiedlichen Wellenlängenbereiche emittieren, insbesondere die im Vorstehenden genannten Wellenlängenbereich, sowie eine Schaltvorrichtung zum wahlweisen Betrieb oder zur wahlweisen Energieversorgung oder zur wahlweisen Strahlungsemission zumindest eines Halbleiterelements, das den ersten Wellenlängenbereich emittiert, oder zumindest eines Halbleiterelements, das den zweiten Wellenlängenbereich emittiert.

Besonders vorteilhafte Ausführungsformen der Erfindung sind in den Unteransprüchen angeführt.

Der Körper der Beleuchtungsvorrichtung oder dessen (Außen-)Mantelfläche oder Außenumfang haben vorzugsweise eine zylindrische Form, insbesondere eine zylindrische Außenform, sie können jedoch auch andere Formen aufweisen, zum Beispiel eckige Formen.

Vorzugsweise weist der Körper der Beleuchtungsvorrichtung eine Oberfläche oder ein Strahlungsabgabeende auf, über welche(s) die von dem zumindest einem Halbleiterelement emittierte elektromagnetische Strahlung von der Beleuchtungsvorrichtung oder in Richtung der Behandlungsstelle abgebbar ist. Die Oberfläche oder das Strahlungsabgabeende weisen bevorzugt entweder eine oder mehrere optisch leitende, insbesondere transparente, vorzugsweise für sichtbares Licht optisch leitende, insbesondere transparente, Strahlungsabgabeflächen auf oder die Oberfläche oder das Strahlungsabgabeende bilden eine Strahlungsabgabefläche. Die Oberfläche, das Strahlungsabgabeende oder die zumindest eine Strahlungsabgabefläche sind vorzugsweise rund oder gebogen, insbesondere um die Aufnahme oder Öffnung, in welcher das Werkzeug aufnehmbar ist, gebogen, oder sie umgeben diese Aufnahme oder Öffnung ring- oder kreisförmig.

Bevorzugt weist der Körper der Beleuchtungsvorrichtung ein Instrumentenanschlussende auf. Das Instrumentenanschlussende ist ausgebildet, die Beleuchtungsvorrichtung an den Instrumentenkopf oder an das Instrument anzulagern oder anzuschließen. Vorzugsweise ist das Instrumentenanschlussende als Übergang oder Schnittstelle zum Übertragen von Medien, zum Beispiel von elektrischer Energie oder eines Fluids, zwischen dem Instrument oder dem Instrumentenkopf und der Beleuchtungsvorrichtung ausgebildet. Alternativ ist das Instrumentenanschlussende als Lager- oder Verbindungsabschnitt für Medienkontaktelemente ausgebildet, zum Beispiel für elektrische Kontakte oder für Fluidkanäle oder Fluidleitungen.

Vorzugsweise erstreckt sich die Mantelfläche des Körpers zwischen dem Strahlungsabgabeende und dem Instrumentenanschlussende des Körpers. Besonders bevorzugt sind das Strahlungsabgabeende und das Instrumentenanschlussende oder die Flächen des Körpers, die das Strahlungsabgabeende und das Instrumentenanschlussende bilden, im Wesentlichen parallel zueinander angeordnet.

Die Öffnung der Beleuchtungsvorrichtung, in welcher das Werkzeug aufnehmbar ist, hat vorzugsweise eine runde oder zylindrische Form, vorzugsweise eine runde oder zylindrisch Innenwand, sie kann jedoch auch andere Formen aufweisen, zum Beispiel eckige Formen.

Die Mantelfläche des Körpers der Beleuchtungsvorrichtung bildet vorzugsweise einen in sich geschlossenen, insbesondere (im Querschnitt) ringförmigen oder mehreckigen, Außenumfang. Die Mantelfläche ist somit nicht unterbrochen oder durchgängig oder weist kein freies Ende auf. Bevorzugt sind zum Zwecke eines kompakten Aufbaus der Beleuchtungsvorrichtung innerhalb der Mantelfläche oder deren Außenumfangs der zumindest eine Fluidkanal, der insbesondere zumindest eine an der Beleuchtungsvorrichtung vorgesehene Öffnung zur Abgabe von Fluid mit einer oder mehreren Fluidquellen verbindet, und / oder die eine oder mehreren optisch leitenden Strahlungsabgabeflächen und / oder die zumindest eine Öffnung zur Abgabe von Fluid und / oder das zumindest eine Halbleiterelement und / oder eine Mischkammer zum Mischen mehrerer Fluide angeordnet. Besonders bevorzugt sind der zumindest eine Fluidkanal und / oder die eine oder mehreren optisch leitenden Strahlungsabgabeflächen und / oder die zumindest eine Öffnung zur Abgabe von Fluid und / oder das zumindest eine Halbleiterelement und / oder eine Mischkammer zum Mischen mehrerer Fluide zwischen der Mantelfläche und der Aufnahme oder Öffnung, in welcher das Werkzeug aufnehmbar ist, angeordnet.

Die Mantelfläche erstreckt sich von einem Strahlungsabgabeende des Körpers oder von einer Oberfläche des Körpers mit einer optisch leitenden Strahlungsabgabefläche oder mit mehreren optisch leitenden Strahlungsabgabeflächen oder zwischen dem Strahlungsabgabeende und dem Instrumentenanschlussende des Körpers.

Vorzugsweise sind in der Beleuchtungsvorrichtung oder in dem Körper der Beleuchtungsvorrichtung mehrere Fluidkanäle vorgesehen, um der Beleuchtungsvorrichtung oder der Behandlungsstelle zum Beispiel unterschiedliche Fluide zuführen zu können, insbesondere ein gasförmiges Fluid, zum Beispiel Luft, und ein flüssiges Fluid, zum Beispiel Wasser oder eine wässrige Lösung. Alternativ können in den mehreren Fluidkanälen auch gleiche Fluide gefördert werden, die jedoch unterschiedliche Eigenschaften haben, zum Beispiel unterschiedliche Temperaturen, unterschiedliche Konzentrationen, unterschiedliche Drücke, unterschiedliche Volumenströme etc. Vorzugsweise können die mehreren Fluidkanäle mit unterschiedlichen Fluidquellen verbindbar oder verbunden sein.

Besonders bevorzugt münden die mehreren Fluidkanäle in eine gemeinsame Fluidmischkammer in der Beleuchtungsvorrichtung oder vereinigen sich zu einer gemeinsamen Fluidmischkammer, wobei die Fluidmischkammer mit der zumindest einen Öffnung zur Abgabe von Fluid verbunden ist. Somit sind in vorteilhafter Weise über die zumindest eine Öffnung zur Abgabe von Fluid Fluidgemische abgebbar, zum Beispiel ein Luft-Wassergemisch.

Die zumindest eine Öffnung zur Abgabe von Fluid kann vorzugsweise als Düse oder Düsenöffnung ausgebildet sein oder eine Düse oder Düsenöffnung aufweisen.

Bevorzugt sind in der Beleuchtungsvorrichtung oder in dem Körper der Beleuchtungsvorrichtung mehrere Fluidkanäle vorgesehen, wobei zumindest ein Fluidkanal eine Öffnung zur Verbindung mit einer Fluidquelle an der (Außen-)Mantelfläche oder am Außenumfang des Körpers der Beleuchtungsvorrichtung und zumindest ein Fluidkanal eine Öffnung zur Verbindung mit einer Fluidquelle an einem Instrumentenanschlussende des Körpers der Beleuchtungsvorrichtung aufweist. In vorteilhafter Weise wird damit, insbesondere wenn die Fluidkanäle sich vereinigen, eine besonders gute Vermischung der in den einzelnen Fluidkanälen geförderten Fluide erzielt.

Vorzugsweise sind der zumindest eine Fluidkanal der Beleuchtungsvorrichtung und / oder die zumindest eine Öffnung zur Abgabe von Fluid der Beleuchtungsvorrichtung über Leitungen und / oder Kanäle im Instrument oder im Instrumentenkopf mit einer oder mehreren Fluidquellen verbunden oder verbindbar.

Vorzugsweise weist die Beleuchtungsvorrichtung mehrere optisch leitende, bevorzugt transparente, insbesondere für sichtbares Licht optisch leitende, bevorzugt transparente, Strahlungsabgabeflächen und mehrere Öffnungen zur Abgabe von Fluid auf, wobei zwischen zwei Öffnungen zur Abgabe von Fluid zumindest eine Strahlungsabgabefläche angeordnet ist. Damit wird in vorteilhafter Weise eine besonders dichte, gleichmäßige und effektive Beleuchtung und Fluidbeaufschlagung der Behandlungsstelle erzielt. Bevorzugt sind zwischen drei und neun, insbesondere fünf, Strahlungsabgabeflächen vorgesehen. Bevorzugt sind zwischen drei und neun, insbesondere fünf, Öffnungen zur Abgabe von Fluid vorgesehen.

Die Anzahl der Öffnungen zur Abgabe von Fluid und der Strahlungsabgabeflächen ist entweder gleich oder unterschiedlich. Die Anzahl der Öffnungen zur Abgabe von Fluid ist gemäß einem Ausführungsbeispiel größer als die Anzahl der Strahlungsabgabeflächen. Gemäß einem alternativen Ausführungsbeispiel ist die Anzahl der Strahlungsabgabeflächen größer als die Anzahl der Öffnungen zur Abgabe von Fluid.

Gemäß einem Ausführungsbeispiel ist zwischen zwei Öffnungen zur Abgabe von Fluid eine Strahlungsabgabefläche vorgesehen. Gemäß einem alternativen Ausführungsbeispiel sind zwischen zwei Öffnungen zur Abgabe von Fluid mehrere Strahlungsabgabefläche vorgesehen. Gemäß einem anderen Ausführungsbeispiel ist zwischen zwei Strahlungsabgabeflächen eine Öffnung zur Abgabe von Fluid vorgesehen. Gemäß einem weiteren Ausführungsbeispiel sind zwischen zwei Strahlungsabgabeflächen mehrere Öffnungen zur Abgabe von Fluid vorgesehen. Gemäß einem Ausführungsbeispiel sind die Öffnungen zur Abgabe von Fluid und die Strahlungsabgabefläche alternierend angeordnet.

Gemäß einem Ausführungsbeispiel sind die zumindest eine Öffnung zur Abgabe von Fluid und die zumindest eine Strahlungsabgabefläche im Wesentlichen gleich weit von der Öffnung, in welcher das Werkzeug aufnehmbar ist, oder von einer Mittelachse dieser Öffnung beabstandet. Gemäß einem alternativen Ausführungsbeispiel sind die zumindest eine Öffnung zur Abgabe von Fluid und die zumindest eine Strahlungsabgabefläche unterschiedlich weit von der Aufnahme oder Öffnung, in welcher das Werkzeug aufnehmbar ist, oder von einer Mittelachse dieser Aufnahme oder Öffnung beabstandet. Gemäß einem Ausführungsbeispiel ist die zumindest eine Öffnung zur Abgabe von Fluid näher an der Öffnung, in welcher das Werkzeug aufnehmbar ist, oder an einer Mittelachse dieser Öffnung angeordnet als die zumindest eine Strahlungsabgabefläche. Gemäß einem alternativen Ausführungsbeispiel ist die zumindest eine Strahlungsabgabefläche näher an der Öffnung, in welcher das Werkzeug aufnehmbar ist, oder an einer Mittelachse dieser Öffnung angeordnet als die zumindest eine Öffnung zur Abgabe von Fluid.

Gemäß einem Ausführungsbeispiel sind die Öffnung zur Abgabe von Fluid und die zumindest eine Strahlungsabgabefläche nebeneinander, insbesondere direkt aneinander grenzend, angeordnet. Gemäß einem alternativen Ausführungsbeispiel ist die Öffnung zur Abgabe von Fluid innerhalb einer Strahlungsabgabefläche angeordnet oder ist die Öffnung zur Abgabe von Fluid von einer Strahlungsabgabefläche umgeben. Gemäß einem anderen Ausführungsbeispiel sind die Öffnung zur Abgabe von Fluid und die Strahlungsabgabefläche voneinander beabstandet, insbesondere durch einen Bereich voneinander beabstandet, der keine elektromagnetische Strahlung, insbesondere keine sichtbare elektromagnetische Strahlung, und kein Fluid abgibt.

Vorzugsweise ist die Beleuchtungsvorrichtung als Ringlicht oder ringförmige Beleuchtungsvorrichtung ausgebildet, bei denen mehrere Halbleiterelemente, die zur Emission von elektromagnetischer Strahlung ausgebildet sind, oder die eine oder mehrere Strahlungsabgabeflächen im Wesentlichen ringförmig um das an den Instrumentenkopf oder das Instrument anschließbare Werkzeug oder um die Werkzeugaufnahmeöffnung des Instrumentenkopfs oder des Instruments oder um die Werkzeughalterung des Instrumentenkopfs oder des Instruments oder um die Öffnung der Beleuchtungsvorrichtung, in welcher das Werkzeug aufnehmbar ist, angeordnet sind. Besonders bevorzugt sind auch die Öffnungen zur Abgabe von Fluid ringförmig um zumindest ein der im Vorstehenden genannten Elemente angeordnet.

Vorzugsweise sind an der Beleuchtungsvorrichtung mehrere elektrisch seriell geschaltete, mittels eines elektrischen Leiters verbundene Halbleiterelemente vorgesehen. Damit sind in vorteilhafter Weise für die elektrische Versorgung der Halbleiterelemente an der Außenseite oder Oberfläche der Beleuchtungsvorrichtung nur zwei elektrische Kontakte vorzusehen. Der elektrischen Leiter ist zum Beispiel als metallische Leiterbahn oder als metallischer Draht ausgebildet. Besonders bevorzugt ist der elektrische Leiter beabstandet von dem zumindest einen in dem Körper der Beleuchtungsvorrichtung vorgesehenen Fluidkanal angeordnet, insbesondere von einem eine Flüssigkeit leitenden Fluidkanal. Insbesondere ist zwischen dem elektrischen Leiter und dem Fluidkanal ein elektrisch isolierendes Material vorgesehen, zum Beispiel ein keramisches Material. Gemäß einem alternativen Ausführungsbeispiel sind zumindest zwei Halbleiterelemente elektrisch parallel geschaltet.

Vorzugsweise ist die Beleuchtungsvorrichtung aus mehreren Schichten, insbesondere aus mehreren Schichten unterschiedlicher Materialien, aufgebaut. Die Materialien, aus denen eine oder mehrere der Schichten aufgebaut sind, umfassen zum Beispiel Glas, Kunststoff, Keramik oder Metall.

Bevorzugt dienen das Glas und / oder der Kunststoff zum optischen Leiten der von dem zumindest einem Halbleiterelement emittierten elektromagnetischen Strahlung durch die Beleuchtungsvorrichtung und / oder an die zumindest eine Strahlungsabgabefläche der Beleuchtungsvorrichtung. Besonders bevorzugt besteht die zumindest eine Strahlungsabgabefläche oder zumindest ein Teil des Strahlungsabgabeendes der Beleuchtungsvorrichtung oder jene Schicht, die eine Strahlungsabgabefläche oder zumindest ein Teil des Strahlungsabgabeendes bildet, aus Glas oder Kunststoff. Besonders bevorzugt emittiert das zumindest eine Halbleiterelement sichtbare Strahlung und sind das Glas und / oder der Kunststoff, die die sichtbare Strahlung optisch leiten, für sichtbare Strahlung transparent.

Vorzugsweise umfassen jene Schicht oder Schichten ein keramisches Material und / oder Kunststoff, in welchen das zumindest eine Halbleiterelement und / oder ein elektrischer Leiter zur Versorgung des zumindest einen Halbleiterelements mit elektrischer Energie vorgesehen ist / sind. Bevorzugt besteht die Schicht, welche einen elektrischen Leiter aufweist, aus zumindest zwei keramischen Teilschichten, zwischen denen der elektrische Leiter angeordnet ist. Besonders bevorzugt weist der zwischen den keramischen Teilschichten angeordnete Leiter keinen elektrisch isolierenden Außenmantel oder keine elektrische Außenisolierung auf. Das keramische Material umfasst zum Beispiel Aluminiumoxid-, Siliciumnitrid- oder Aluminiumnitridkeramiken.

Vorzugsweise umfasst zumindest eine der Schichten metallisches Material und / oder Kunststoff und / oder Glas, insbesondere jene Schicht, die das Strahlungsabgabeende der Beleuchtungsvorrichtung bildet oder jene Schicht, an welcher die zumindest eine optisch leitenden Strahlungsabgabefläche vorgesehen ist. Das metallische Material umfasst zum Beispiel Stahl. Der Kunststoff umfasst zum Beispiel Polymethylmethacrylat (PMMA).

Ein Ausführungsbeispiel einer Beleuchtungsvorrichtung umfasst eine keramische Schicht, an welcher das zumindest eine Halbleiterelement und / oder der elektrischer Leiter zur Versorgung des zumindest einen Halbleiterelements vorgesehen ist / sind, und eine damit verbundene metallische Schicht, in welcher zumindest ein optischer Leiter aus Glas und / oder zumindest eine Strahlungsabgabefläche aus Glas zum Leiten der von dem Halbleiterelement emittierte elektromagnetische, insbesondere sichtbare, Strahlung angeordnet ist / sind. Vorzugsweise ist das Glas in die metallische Schicht eingeschmolzen.

Ein alternatives Ausführungsbeispiel einer Beleuchtungsvorrichtung umfasst eine keramische Schicht, an welcher das zumindest eine Halbleiterelement und / oder der elektrischer Leiter zur Versorgung des zumindest einen Halbleiterelements vorgesehen ist / sind, und eine damit verbundene Glasschicht zum Leiten der von dem Halbleiterelement emittierte elektromagnetische, insbesondere sichtbare, Strahlung. Dieses Ausführungsbeispiel weist keine metallische Schicht auf.

Ein anderes Ausführungsbeispiel einer Beleuchtungsvorrichtung umfasst eine erste metallische Schicht, an welcher das zumindest eine Halbleiterelement und / oder der elektrischer Leiter zur Versorgung des zumindest einen Halbleiterelements vorgesehen ist / sind, gegebenenfalls von der metallischen Schicht durch ein elektrisches Isolationsmittel getrennt, und eine damit verbundene zweite metallische Schicht, in welcher zumindest ein optischer Leiter aus Glas und / oder zumindest eine Strahlungsabgabefläche aus Glas angeordnet ist / sind oder eine damit verbundene Glasschicht, so wie dies im Vorstehenden beschrieben ist.

Vorzugsweise weist die Beleuchtungsvorrichtung mehrere Halbleiterelemente auf, wobei jedem Halbleiterelement ein eigener optischer Strahlungsleiter zugeordnet ist, der derart angeordnet ist, dass er die von den Halbleiterelementen emittierte elektromagnetische Strahlung in Richtung des Strahlungsabgabeendes der Beleuchtungsvorrichtung oder in Richtung der zumindest einen optisch leitenden Strahlungsabgabefläche leitet. Die Strahlungsleiter erstrecken sich zum Beispiel von den Halbleiterelementen in Richtung des Strahlungsabgabeendes oder der Strahlungsabgabefläche. Zur weiteren Verbesserung der Strahlungs-/Lichtübertragung können die Strahlungsleiter bevorzugt beschichtet sein. Besonders bevorzugt bildet zumindest ein Ende eines Strahlungsleiters zumindest einen Teil der Strahlungsabgabefläche der Beleuchtungsvorrichtung oder zumindest einen Teil des Strahlungsabgabeendes der Beleuchtungsvorrichtung. Die Strahlungsleiter umfassen zum Beispiel einen Glaskörper, Glasstab, Glasfaserkörper, Glasfaserstab, Kunststoffkörper oder Kunststoffstab. Besonders bevorzugt sind die optischen Strahlungsleiter voneinander beabstandet, insbesondere durch ein Material voneinander getrennt, das keine elektromagnetische Strahlung, insbesondere keine sichtbare elektromagnetische Strahlung, abgibt oder das nicht transparent ist, insbesondere nicht für Licht transparent ist.

Vorzugsweise ist das zumindest eine Halbleiterelement in einer, insbesondere hermetisch verschlossenen, Kammer im Körper der Beleuchtungsvorrichtung angeordnet ist. Bevorzugt ist die Kammer derart hermetisch verschlossen, dass keine Partikel und / oder Wasserdampf und / oder Flüssigkeiten in die Kammer eindringen. Besonders bevorzugt ist die Kammer derart hermetisch verschlossen, dass sie mehreren Reinigungs- oder Sterilisationsvorgängen widersteht, so dass in den Vorgängen verwendete Medien, wie Reinigungsmittel oder Wasserdampf, nicht in die Kammer eindringen. Bevorzugt ist zumindest ein Teil der Kammer oder der den Innenraum der Kammer umgebende Kammerwand durch ein Material gebildet, welches für die von dem Halbleiterelement emittierte Strahlung transparent ist und / oder diese Strahlung leitet.

Vorzugsweise ist die Kammer oder die den Innenraum der Kammer umgebende Kammerwand durch mehrere Schichten der Beleuchtungsvorrichtung gebildet, die miteinander verbunden sind, zum Beispiel durch verschmelzen, einschmelzen, löten, verschweißen, verkleben, etc. Gemäß einem Ausführungsbeispiel ist die Kammerwand durch eine Keramikschicht und eine damit verbundene Metallschicht gebildet. Besonders bevorzugt ist in der Metallschicht ein optischer Strahlungsleiter angeordnet, der insbesondere auch einen Teil der Kammerwand bildet. Gemäß einem anderen Ausführungsbeispiel ist die Kammerwand durch eine Keramikschicht und eine damit verbundene Glasschicht gebildet. Gemäß einem weiteren Ausführungsbeispiel ist die Kammerwand durch eine Keramikschicht und eine Metallschicht und eine Glasschicht, die miteinander verbunden sind, gebildet.

Vorzugsweise ist in der Kammer neben dem Halbleiterelement zumindest ein zusätzliches Bauteil vorgesehen. Gemäß einem Ausführungsbeispiel umfasst das zusätzliche Bauteil ein Element zur Umwandlung der Wellenlänge der vom Halbleiterelement abgestrahlten Strahlung, zum Beispiel einen Konverter, eine Konversionsfolie oder eine Konversionspaste. Gemäß einem anderen Ausführungsbeispiel umfasst das zusätzliche Bauteil einen elektrischen Leiter zur Versorgung des Halbleiterelements mit elektrischer Energie. Gemäß einem anderen Ausführungsbeispiel umfasst das zusätzliche Bauteil ein optisches Element, zum Beispiel einen Reflektor, insbesondere zum Lenken der vom Halbleiterelement abgestrahlten Strahlung in Richtung der Strahlungsabgabefläche und / oder eine Linse zum Bündeln der vom Halbleiterelement abgestrahlten Strahlung und / oder einen optischen Filter.

Bevorzugt schließt an die, insbesondere hermetisch verschlossenen, Kammer ein optischer Strahlungsleiter an, der derart angeordnet ist, dass er die von den Halbleiterelementen emittierte elektromagnetische Strahlung in Richtung des Strahlungsabgabeendes der Beleuchtungsvorrichtung oder in Richtung der zumindest einen optisch leitenden Strahlungsabgabefläche leitet, so wie er insbesondere im Vorstehenden beschrieben ist. Alternativ bildet der optischer Strahlungsleiter oder eine Fläche des optischen Strahlungsleiters einen Teil der den Innenraum der Kammer umschließenden Kammerwand.

Vorzugsweise umfasst die Beleuchtungsvorrichtung eine Steuervorrichtung zur alternierenden Abgabe der elektromagnetischen Strahlung und des Fluids vom Strahlungsabgabeende der Beleuchtungsvorrichtung. Gemäß einem Ausführungsbeispiel ist die Steuervorrichtung ausgebildet, abwechselnd einen oder mehrere Strahlungspulse und anschließend einen Fluidpuls von der Beleuchtungsvorrichtung abzugeben, die im Wesentlichen zeitlich aufeinander folgen. Gemäß einem alternativen bevorzugten Ausführungsbeispiel ist die Steuervorrichtung ausgebildet, abwechselnd einen oder mehrere Fluidpulse und anschließend einen Strahlungspuls von der Beleuchtungsvorrichtung abzugeben, die im Wesentlichen zeitlich aufeinanderfolgen.

Die Steuervorrichtung umfasst bevorzugt ein oder mehrere auf die Lichtabgabevorrichtung oder auf die Kühlmedienabgabevorrichtung einwirkende Steuer- oder Stellelemente und insbesondere einen damit verbundenen Mikrocontroller zur Ansteuerung der Steuer- oder Stellelemente. Besonders bevorzugt weist die Steuervorrichtung ein in einer Medienleitung eines Kühlmediums angeordnetes Steuerventil auf, insbesondere ein Magnetventil, das vom Mikrocontroller zum Öffnen oder Schließen durch Steuersignale periodisch ansteuerbar ist. Die Pulsrate der Steuersignale oder des Steuerventils und somit auch der Kühlmedienabgabe beträgt zumindest in etwa 25 Hz, bevorzugt mehr als etwa 50 Hz, besonders bevorzugt etwa 75 Hz.

Bevorzugt weist die Steuervorrichtung des Weiteren ein elektrisches oder elektronisches Schaltelement zur gepulsten Energieversorgung (Versorgung mit elektrischem Strom) des zumindest einen Halbleiterelements auf. Die Pulsrate der Beleuchtungsvorrichtung liegt bevorzugt über der Flimmerfrequenz des menschlichen Auges und beträgt zumindest in etwa 25 Hz, besonders bevorzugt mehr als etwa 50 Hz, so dass für den Anwender der Eindruck einer kontinuierlichen Lichtabgabe entsteht.

Vorzugsweise ist die Beleuchtungsvorrichtung lösbar mit dem medizinischen, insbesondere dentalen, Instrument oder Instrumentenkopf verbindbar. Die lösbare Verbindung erfolgt zum Beispiel mittels einer Steck-, Klemm- oder Schraubverbindung. Vorzugsweise ist an der Beleuchtungsvorrichtung ein Anschlag, zum Beispiel ein Flansch, vorgesehen, der ausgebildet ist, an einem Gegenanschlag des Instrumentenkopfs anzuliegen, sobald die Beleuchtungsvorrichtung ihre vornestimmte Position am Instrumentenkopf einnimmt.

Vorzugsweise ist an dem Instrumentenkopf eine Aufnahme, zum Beispiel eine Rücksprung, vorgesehen, in dem die Beleuchtungsvorrichtung, insbesondere lösbar, aufnehmbar ist. Bevorzugt ist die Aufnahme ausgebildet, Fluid an die Beleuchtungsvorrichtung zu übertragen. Besonders bevorzugt ist diese Aufnahme mit zumindest einer Fluidleitung des Instrumentenkopfs oder des Instruments verbunden oder mündet eine Fluidleitung in die Aufnahme, so dass Fluid von einer Fluidquelle über die Fluidleitung und über oder durch die Aufnahme zur Beleuchtungsvorrichtung oder zu dem zumindest einen Fluidkanal der Beleuchtungsvorrichtung oder zur Fluidabgabeöffnung der Beleuchtungsvorrichtung förderbar ist. Alternativ oder zusätzlich dient zumindest ein Teil der Aufnahme als Fluidkanal, welcher der Beleuchtungsvorrichtung ein Fluid zuführt oder bildet zumindest ein Teil der Aufnahme gemeinsam mit der Beleuchtungsvorrichtung, zum Beispiel einer Ausnehmung der Beleuchtungsvorrichtung, zumindest einen Abschnitt des Fluidkanals der Beleuchtungsvorrichtung.

Gemäß einem Ausführungsbeispiel ist die Beleuchtungsvorrichtung elektrisch mit einem in dem medizinischen, insbesondere dentalen, Instrument angeordneten Generator verbunden, so dass die Beleuchtungsvorrichtung, insbesondere das zumindest eine Halbleiterelement, mit von dem Generator erzeugter elektrischer Energie versorgbar ist. Der Generator ist zur Erzeugung der elektrischen Energie vorzugsweise durch ein Antriebselement des Instruments, das ausgebildet ist, die Werkzeughaltevorrichtung oder ein mit dem Instrument verbindbares Werkzeug in Bewegung zu versetzen, antreibbar, zum Beispiel durch eine Antriebswelle. Alternativ ist der Generator durch ein Fluid, insbesondere durch ein Antriebsfluid für die Werkzeughaltevorrichtung oder ein mit dem Instrument verbindbares Werkzeug, antreibbar.

Vorzugsweise ist der Beleuchtungsvorrichtung ein elektrischer oder elektronischer Steuer- oder Schaltkreis zugeordnet oder es ist in dem medizinischen, insbesondere dentalen, Instrument ein elektrischer oder elektronischer Steuer- oder Schaltkreis vorgesehen, der ausgebildet ist, zumindest einen Parameter der elektrischen Energie zur Versorgung des zumindest einen Halbleiterelements, insbesondere die elektrische Spannung, an das Halbleiterelement anzupassen. Mittels des Steuer- oder Schaltkreises ist alternativ oder zusätzlich zum Beispiel auch die Helligkeit der von dem Halbleiterelement emittierten Strahlung einstellbar oder es ist aus mehreren Halbleiterelementen wahlweise ein Halbleiterelement mit einer bestimmten Eigenschaft, zum Beispiel mit einem spezifischen Wellenlängenbereich, wie im Vorstehenden bereits beschrieben, betreibbar oder mit Energie versorgbar.

Die Erfindung wird nachfolgend anhand bevorzugter Ausführungsbeispiele und Bezug nehmend auf die beigefügten Zeichnungen erläutert:
Figur 1 zeigt ein Ausführungsbeispiel eines medizinischen, insbesondere dentalen, Instruments mit einer Beleuchtungsvorrichtung mit zumindest einem Halbleiterelement zur Emission von elektromagnetischer, insbesondere sichtbarer, Strahlung und zumindest einem in der Beleuchtungsvorrichtung vorgesehenen Fluidkanal, der zumindest eine am Strahlungsabgabeende der Beleuchtungsvorrichtung vorgesehene Öffnung zur Abgabe von Fluid mit einer oder mehreren Fluidquellen verbindet.
Figur 2 zeigt ein Ausführungsbeispiel eines Strahlungsabgabeendes einer Beleuchtungsvorrichtung mit zumindest einem Halbleiterelement, wobei das Strahlungsabgabeende mehrere Strahlungsabgabeflächen und mehrere Öffnung zur Abgabe von Fluid aufweist.
Figur 3 einen Querschnitt durch die Beleuchtungsvorrichtung der Figur 2 entlang der Linie "A-A".
Figur 4 zeigt den in der Figur 3 mit "B" bezeichnet Ausschnitt in vergrößertem Maßstab mit einem Halbleiterelement und einem dem Halbleiterelement zugeordneten optischen Leiter.
Figur 5 zeigt einen Längsschnitt durch die Beleuchtungsvorrichtung der Figur 3 entlang der Linie "C - C" sowie die elektrische Versorgung der Halbleiterelemente.
Figur 6 zeigt einen Querschnitt durch eine alternative Ausführungsform einer Beleuchtungsvorrichtung mit zumindest einem Halbleiterelement und zumindest einer Öffnung zur Abgabe von Fluid.
Figur 7 zeigt ein weiteres Ausführungsbeispiel einer Kammer einer Beleuchtungsvorrichtung mit einem Halbleiterelement und einem dem Halbleiterelement zugeordneten optischen Leiter.
Figur 8 zeigt ein alternatives Ausführungsbeispiel eines Strahlungsabgabeendes einer Beleuchtungsvorrichtung mit zumindest einem Halbleiterelement, wobei das Strahlungsabgabeende eine Strahlungsabgabefläche aufweist, in der mehrere Öffnungen zur Abgabe von Fluid angeordnet sind.
Figur 9 zeigt ein Ausführungsbeispiel eines Instrumenten- oder Winkelstückkopfs mit einer damit lösbar verbundenen Beleuchtungsvorrichtung mit zumindest einem Halbleiterelement und zumindest einer Öffnung zur Abgabe von Fluid sowie in vergrößerten Ausschnitten ein Halbleiterelement der Beleuchtungsvorrichtung und einen Fluidkanal der Beleuchtungsvorrichtung.
Figur 10 zeigt ein alternatives Ausführungsbeispiel einer Beleuchtungsvorrichtung mit einem Ringkanal für ein Fluid an der Mantelfläche.
Figur 11 zeigt ein alternatives Ausführungsbeispiel eines Instrumenten- oder Winkelstückkopfs mit einer damit unlösbar verbundenen Beleuchtungsvorrichtung.
Figur 12 zeigt ein alternatives Ausführungsbeispiel einer Beleuchtungsvorrichtung, deren Körper aus Kunstharz besteht, in welchem das zumindest eine Halbleiterelement vergossen ist.
Figur 13 zeigt ein alternatives Ausführungsbeispiel einer Beleuchtungsvorrichtung mit zwei freien Enden.
Figur 14 zeigt ein alternatives Ausführungsbeispiel einer Beleuchtungsvorrichtung, deren Körper aus zwei Teilringen besteht.

Die Figuren 15 und 16 zeigen zwei Ausführungsbeispiele von Beleuchtungsvorrichtungen, deren Sockel aus keramischem Material oder aus glaskeramischem Material oder aus Glas durch ein Metall und Glas aufweisendes Material mit hohlen, metallischen Hülsen verbunden sind.

Die Figuren 17 und 18 zeigen die Oberseite und Unterseite eines Sockels aus keramischem Material oder aus glaskeramischem Material oder aus Glas einer Beleuchtungsvorrichtung sowie die darauf vorgesehenen elektrischen Kontakte.

Figur 19 zeigt eine Vergrößerung des in der Figur 15 mit "A" gekennzeichneten Ausschnitts.

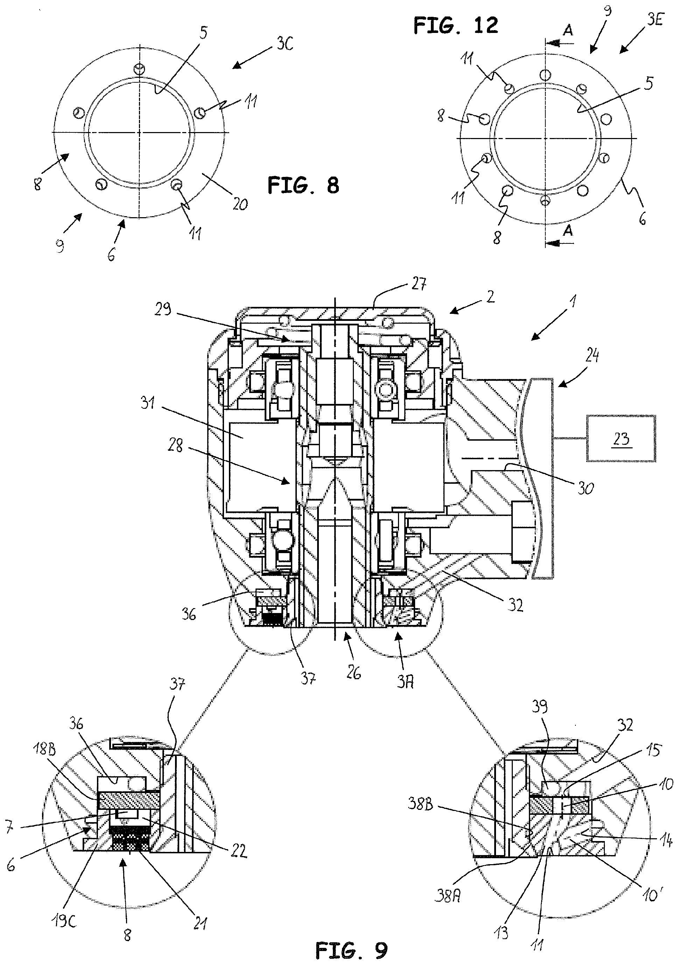

Das in den Figuren 1 und 9 dargestellte medizinische, insbesondere dentale, Instrument 1 ist als längliches, rohrförmiges Instrument 1 oder Handstück ausgebildet, das an einem Ende einen Anschluss 24, zum lösbaren Verbinden zum Beispiel an eine Steuervorrichtung, an ein Antriebseinheit, an eine Energiequelle und / oder an eine Fluidquelle, insbesondere eine Wasser- und / oder Druckluftquelle, aufweist. Das Instrument 1 umfasst einen gebogenen oder einen zwei gewinkelt zueinander angeordnete Abschnitte aufweisenden Griffteil 25 und einen daran anschließenden Instrumentenkopf 2. Am Instrumentenkopf 2 ist eine Werkzeugöffnung 26 vorgesehen, durch die ein Werkzeug 4 zum Einwirken auf eine Behandlungsstelle lösbar in den Instrumentenkopf 2 einbringbar ist. Im Instrumentenkopf 2 ist eine lösbare Werkzeughaltevorrichtung 28, zum Beispiel eine Spannzange, angeordnet, welche das Werkzeug 4 lösbar am Instrumentenkopf 2 befestigt. Die Werkzeugöffnung 26 ist seitlich am Instrumentenkopf 2 angeordnet, so dass das Werkzeug 4 gewinkelt zum Griffteil 25 oder dessen Längsachse aus dem Instrumentenkopf 2 ragt. An dem der Werkzeugöffnung 26 gegenüberliegenden Ende des Instrumentenkopfs 2 ist ein Drucktaster 27 vorgesehen, der mit einer im Instrumentenkopf 2 angeordneten Werkzeuglösevorrichtung 29 zusammenwirkt, um das Werkzeug 4 aus dem Instrumentenkopf 2 oder der Werkzeughaltevorrichtung 28 frei zu geben. Selbstverständlich kann das Instrument 1 auch andere bekannte Formen aufweisen, zum Beispiel pistolenförmig oder gerade ausgebildet sein.

Von der Anschlussvorrichtung 24 erstrecken sich zum Beispiel eine Vorrichtung zur Übertragung von Antriebsenergie auf die Werkzeughaltevorrichtung 28, insbesondere eine Welle oder eine Fluidleitung, eine oder mehrere Fluid- oder Medienleitungen 32, zum Beispiel eine oder mehrere Medienleitungen für (Kühl-)Wasser oder (Kühl-)Druckluft, Lichtleiter und / oder elektrische Versorgungs- oder Steuerleitungen durch das Instrument 1. Gemäß der Figur 9 fördert eine Fluidleitung 30 ein Antriebsfluid, zum Beispiel Druckluft, zu einer Antriebseinheit in Form eines in Drehung versetzbaren Drehteils 31, insbesondere eines Laufrads. Das Drehteil 31 ist mit der Werkzeughaltevorrichtung 28 verbunden, um diese und ein darin aufgenommenes Werkzeug 4 in Bewegung zu versetzen.

An dem Instrumentenkopf 2, insbesondere an dem Ende des Instrumentenkopfs 2 mit der Werkzeugöffnung 26, ist eine Beleuchtungsvorrichtung 3, 3A vorgesehen. Die Beleuchtungsvorrichtung 3, 3A ist insbesondere um die Werkzeugöffnung 26 angeordnet oder umgibt diese ringförmig, so dass die Beleuchtungsvorrichtung 3, 3A als Ringlicht ausgebildet ist.

Die Figuren 2 - 4 zeigen ein Ausführungsbeispiel einer Beleuchtungsvorrichtung 3: Die Beleuchtungsvorrichtung 3 weist ein Strahlungsabgabeende 9 und ein im Wesentlichen parallel dazu angeordnetes Instrumentenanschlussende 16 auf. Vom Strahlungsabgabeende 9 emittiert die Beleuchtungsvorrichtung 3 elektromagnetische Strahlung, insbesondere sichtbare elektromagnetische Strahlung. Das Instrumentenanschlussende 16 dient zum Anlagern oder Verbinden der Beleuchtungsvorrichtung 3 an das Instrument 1. Zwischen den beiden Enden 9, 16 erstreckt sich ein Körper 6 der Beleuchtungsvorrichtung 3 mit einer (Außen-)Mantelfläche 12. Die Mantelfläche 12 verbindet somit die beiden Enden 9, 16. Die Mantelfäche 12 ist in sich geschlossen oder weist einen geschlossenen, insbesondere zylindrischen, Außenumfang auf.

Der Körper 6 der Beleuchtungsvorrichtung 3 weist eine, insbesondere zentrale, Bohrung oder Aufnahme oder Öffnung 5 auf. Die Öffnung 5 ist fluchtend an die Werkzeugöffnung 26 angeschlossen oder umgibt die Werkzeugöffnung 26. In der Aufnahme oder Öffnung 5 ist das Werkzeug 4 aufnehmbar oder durch die Aufnahme oder Öffnung 5 ist das Werkzeug 4 in den Instrumentenkopf 2 einbringbar oder daraus lösbar. Wie aus der Figur 2 erkennbar ist, kann die Bohrung oder Öffnung 5 zylindrisch ausgebildet sein.

An oder in dem Körper 6 ist ein Halbleiterelement 7 vorgesehen, das zur Emission von elektromagnetischer Strahlung, insbesondere von sichtbarer Strahlung (Licht), ausgebildet ist. Das Halbleiterelement 7 ist zum Beispiel als Leuchtdiode (LED) oder als Dice ausgebildet. Zumindest ein Teil der von dem Halbleiterelement 7 erzeugten Strahlung wird über eine oder mehrere optisch leitende, insbesondere transparente, Licht- oder Strahlungsabgabeflächen 8 an dem Strahlungsabgabeende 9 der Beleuchtungsvorrichtung 3 oder an einer Oberfläche der Beleuchtungsvorrichtung 3 abgegeben.

In dem Körper 6 der Beleuchtungsvorrichtung 3 ist des Weiteren zumindest ein Fluidkanal 10, 10' vorgesehen, der von dem Strahlungsabgabeende 9 oder der Oberfläche mit der Strahlungsabgabefläche 8 durch zumindest einen Teil der Beleuchtungsvorrichtung 3 verläuft. Der Fluidkanal 10, 10' endet mit einer Öffnung 11 zur Abgabe von Fluid am Strahlungsabgabeende 9 oder der Oberfläche mit der Licht- oder Strahlungsabgabefläche 8. Der zumindest eine Fluidkanal 10, 10' ist mit der Fluidleitung 32 verbunden (siehe Figur 9) und ausgebildet, zumindest ein Fluid aus der Fluidleitung 32 durch die Beleuchtungsvorrichtung 3 bis zur Öffnung 11 zu leiten. Aus der Öffnung 11 wird das Fluid an die Umgebung abgegeben, zum Beispiel auf die Behandlungsstelle und / oder das Werkzeug 4.

Der zumindest eine Fluidkanal 10, 10', das zumindest eine Halbleiterelement 7, die Öffnung 11 und die Licht- oder Strahlungsabgabefläche 8 sind innerhalb der in sich geschlossenen Mantelfläche 12 des Körpers 6 oder des in sich geschlossenen Außenumfangs der Mantelfläche 12 angeordnet.

Das Ausführungsbeispiel gemäß Figur 2 zeigt ein Strahlungsabgabeende 9 einer Beleuchtungsvorrichtung 3 mit mehreren, insbesondere jeweils fünf, Öffnungen 11 zur Abgabe von Fluid und Licht- oder Strahlungsabgabeflächen 8. Die Öffnungen 11 und die Strahlungsabgabeflächen 8 sind jeweils abwechselnd angeordnet. Zwischen zwei Öffnungen 11 ist eine Strahlungsabgabefläche 8 bzw. zwischen zwei Strahlungsabgabeflächen 8 ist eine Öffnung 11 vorgesehen. Die Öffnungen 11 und die Strahlungsabgabeflächen 8 umgeben die Aufnahme oder Öffnung 5 im Wesentlichen kreisförmig. Die Öffnungen 11 sind etwas näher an der Aufnahme oder Öffnung 5 positioniert als die Strahlungsabgabeflächen 8. Die Öffnungen 11 und die Strahlungsabgabeflächen 8 sind voneinander beabstandet. Die Abschnitte oder Flächen des Strahlungsabgabeendes 9 zwischen den Öffnungen 11 und den Strahlungsabgabeflächen 8 sind nicht dazu ausgebildet, Fluid und / oder Strahlung abzugeben, insbesondere sind diese Flächen im Wesentlichen nicht optisch leitend oder nicht transparent für sichtbares Licht. Die Flächen sind zum Beispiel aus Metall, insbesondere aus Stahl hergestellt. Selbstverständlich ist jedoch auch eine andere Anzahl und / oder Anordnung der Öffnungen 11 und / oder der Strahlungsabgabeflächen 8 möglich.

Aus der Figur 3 ist zu erkennen, dass die Beleuchtungsvorrichtung 3 zumindest zwei Fluidkanäle 10, 10' aufweist. Vorzugsweise sind diese beiden Fluidkanäle 10, 10' mit unterschiedlichen Fluidleitungen 32 des Instruments 1 verbunden, insbesondere mit unterschiedlichen Fluidquellen, zum Beispiel mit einer Wasserquelle und einer Druckluftquelle. Der Fluidkanal 10' weist eine Öffnung 14 zur Verbindung mit einer Fluidquelle an der Mantelfläche 12 des Körpers 6 auf, der Fluidkanal 10 eine Öffnung 15 zur Verbindung mit einer Fluidquelle an dem Instrumentenanschlussende 16 des Körpers 6 auf.

Vorzugsweise vereinigen sich die beiden Fluidkanäle 10, 10' zu einer Fluidmischkammer 13 oder münden in eine Fluidmischkammer 13, in welcher sich die Fluide aus den beiden Fluidkanälen 10, 10' vermischen. Die Fluidmischkammer 13 und über die Fluidmischkammer 13 auch die beiden Fluidkanäle 10, 10' sind mit zumindest einer Öffnung 11 zur Abgabe von Fluid verbunden, über welche die beiden, bevorzugt vermischten, Fluide oder das Fluidgemisch von der Beleuchtungsvorrichtung 3, insbesondere von deren Strahlungsabgabeende 9, abgegeben werden.

Insbesondere aus der Figur 4 ist ersichtlich, dass das zumindest eine Halbleiterelement 7 in einer, insbesondere hermetisch verschlossenen, Kammer 22 aufgenommen ist. Bei Vorhandensein mehrerer Halbleiterelemente 7 sind vorzugsweise auch mehrere voneinander getrennte Kammern 22 vorgesehen, insbesondere ist in jeder Kammer 22 nur jeweils ein Halbleiterelement 7 aufgenommen (siehe Figur 5). Die Kammer 22 schützt das Halbleiterelement 7 vor Kontamination, insbesondere vor Partikeln oder flüssigen, gas- oder dampfförmigen Verunreinigungen.

Die Kammer 22 ist innerhalb der Mantelfläche 12 des Körpers 6 angeordnet. Die Kammer 22 oder die Innenwände der Kammer 22 werden vorzugsweise durch mehrere Schichten 18, 19, aus denen der Körper 6 der Beleuchtungsvorrichtung 3 besteht, gebildet.

In der Kammer 22 ist bevorzugt neben dem Halbleiterelement 7 zumindest ein zusätzliches Bauteil vorgesehen. Wie insbesondere in der Figur 4 zu erkennen ist, umfasst das zusätzliche Bauteil ein Element 33 zur Umwandlung der Wellenlänge der vom Halbleiterelement abgestrahlten Strahlung, insbesondere zum Umwandeln in weißes Licht, zum Beispiel einen Konverter, insbesondere eine Konversionsfolie oder eine Konversionspaste. Solche Konverter können Farbkonversionsmaterialien enthalten, beispielsweise Lumineszenzfarbstoffe, die insbesondere durch blaues Licht zum Leuchten angeregt werden, wodurch langwelligeres, gelbes Licht abgegeben wird. Da nicht das gesamte blaue Licht umgewandelt wird, gibt die resultierende additive Mischung der Spektralfarben ein weißes Licht. Als Farbkonversionsmaterialien können beispielsweise Orthosilikate oder YAG Farbstoffe eingesetzt werden, die Farbkonversionspigmente können insbesondere in einem organischen Trägermaterial, bevorzugt transparenter Epoxidharz oder silikonbasierte Trägermaterialen eingebettet sein.

Die Beleuchtungsvorrichtung 3 weist des Weiteren zumindest einen optischen Strahlungsleiter 21 auf, der derart angeordnet ist, dass er von dem Halbleiterelement 7 emittierte elektromagnetische Strahlung in Richtung des Strahlungsabgabeendes 9 und / oder der Licht- oder Strahlungsabgabefläche 8 der Beleuchtungsvorrichtung 3 leitet. Sind mehrere Halbleiterelement 7 vorgesehen, so können auch mehrere Strahlungsleiter 21 vorhanden sein, vorzugsweise ist jedem Halbleiterelement 7 ein eigener Strahlungsleiter 21 zugeordnet. Der Strahlungsleiter 21 weist zwei Enden 21A, 21B auf, wobei ein erstes Ende 21A einem Halbleiterelement 7 zugewandt ist. Das erste Ende 21A kann an die Kammer 22 anschließen oder einen Teil der Begrenzung der Kammer 22 bilden, insbesondere einen Teil der Begrenzungswand der Kammer 22. Das zweite Ende 21B des Strahlungsleiters 21 weist in Richtung des Strahlungsabgabeendes 9 oder bildet zumindest einen Teil der Licht- oder Strahlungsabgabefläche 8 der Beleuchtungsvorrichtung 3. Der Strahlungsleiter 21 ist zum Beispiel als Zylinder, Stab (siehe Figur 4) oder flache Scheibe (siehe Figur 6) ausgebildet. Der Strahlungsleiter 21 ist insbesondere in einer Bohrung der Beleuchtungsvorrichtung 3 oder einer Schicht 18, 19 der Beleuchtungsvorrichtung 3 aufgenommen, zum Beispiel durch Einschmelzen. Der Strahlungsleiter 21 ist zum Beispiel als Glas-, Glasfaserbündel-, Kunstharz- oder Kunststoffelement ausgebildet.

Insbesondere die Figuren 3, 4, 6 und 7 zeigen, dass die Beleuchtungsvorrichtungen 3, 3A und 3B oder der Körper 6 aus mehreren Schichten 18, 19 besteht. Die Schichten 18, 19 sind vorzugsweise fest miteinander verbunden, zum Beispiel durch Verschweißen, Löten, Verschmelzen oder Verkleben.

Die Beleuchtungsvorrichtung 3 der Figuren 3 und 4 weist eine erste keramische Schicht 18 auf, in welcher bevorzugt auch der elektrische Leiter 17 (siehe Figur 5) zur Versorgung des zumindest einen Halbleiterelements 7 angeordnet ist. Vorzugsweise sind auch die elektrischen Kontakte 17A, 17B, die den elektrischen Leiter 17 mit einer elektrischen Energiequelle verbinden an der keramischen Schicht 18 vorgesehen. Die keramische Schicht 18 ist zum Beispiel rund oder kreisförmig oder ringförmig geformt.

An die keramische Schicht 18 schließt eine zweite keramische Schicht 18A an, in welcher zumindest eine Bohrung vorgesehen ist, die zumindest einen Teil der Kammer 22 bildet. Die Wand der Bohrung bildet somit einen Teil der Innenwand der Kammer 22. Daran schließt eine dritte, metallische Schicht 19 an. An dieser metallischen Schicht 19 ist bevorzugt zumindest ein Element 34 zum Anlagern und oder Befestigen der Beleuchtungsvorrichtung 3 an dem Instrumentenkopf 2 vorgesehen, zum Beispiel ein Anschlag, ein Fortsatz, ein Rücksprung, ein Vorsprung, ein Gewinde oder ähnliches. Die metallische Schicht 19 bildet auch das Strahlungsabgabeende 9 oder die Oberfläche der Beleuchtungsvorrichtung 3, an welcher sich die Licht- oder Strahlungsabgabeflächen 8 befinden.

Die drei Schichten 18, 18A und 19 sind insbesondere nicht dazu ausgebildet, sichtbares Licht zu leiten, d.h. sie sind nicht transparent. Zum Leiten der von dem zumindest einen Halbleiterelement 7 erzeugten elektromagnetische Strahlung, insbesondere des sichtbaren Lichts, ist deshalb zumindest ein im Vorstehenden bereits beschriebener optischer Strahlungsleiter 21 vorgesehen. Zur Aufnahme des Strahlungsleiters 21 weist zumindest eine der Schichten 18, 18A, 19 eine Bohrung 35 auf (zum Beispiel gemäß Figur 4 die Schicht 19), die insbesondere von dem Strahlungsabgabeende 9 oder einer Oberfläche der Beleuchtungsvorrichtung 3 bis zur Kammer 22 reicht.

Die Figur 5 zeigt in einer Aufsicht den Verlauf des elektrischen Leiters 17 zur Versorgung der Halbleiterelemente 7 mit elektrischer Energie in der keramischen Schicht 18. Es ist zu erkennen, dass die Halbleiterelemente 7 seriell geschaltet und mittels des elektrischen Leiters 17 verbunden sind. Der elektrische Leiter 17 ist des Weiteren beabstandet von dem Fluidkanal 10 angeordnet. Aufgrund der elektrischen Isolierung durch die keramische Schicht 18 benötigt der Leiter 17 keinen elektrisch isolierenden Außenmantel oder keine elektrische Außenisolierung.

Die Beleuchtungsvorrichtungen 3A und 3B der Figuren 6 und 7 ähneln in ihrem Aufbau der Beleuchtungsvorrichtung 3 der Figuren 2 - 5, wobei zur Vermeidung von Wiederholungen gleiche Bauteile mit den gleichen Bezugszeichen versehen sind.

Abweichend von der Beleuchtungsvorrichtung 3 umfasst die Beleuchtungsvorrichtung 3A der Figur 6 nur zwei Schichten 18B und 19C. Die Schicht 18B ist durch ein keramisches Material gebildet. Die Schicht 18B hat vorzugsweise die Form eines keramischen Rings. Mit der Schicht 18B verbunden ist eine metallische Schicht 19C. Die im Wesentlichen ebenfalls ringförmige Schicht 19C weist zumindest eine Bohrung 35A auf, in welcher der Strahlungsleiter 21 aufnehmbar ist. Vorzugsweise bildet zumindest ein Teil der Bohrung 35A zumindest auch einen Teil der Kammer 22 oder sind das zumindest eine Halbleiterelement 7 und / oder das Konverterelement 33 in der Bohrung 35A aufgenommen.

Die Beleuchtungsvorrichtung 3B der Figur 7 besteht ebenfalls nur aus zwei Schichten 19A, 19B. Diese Schichten 19A, 19B sind vorzugsweise beide aus Metall. Der in der Schicht 19A angeordnete elektrische Leiter 17 ist demgemäß mit einer elektrischen Isolierung versehen. In zumindest einer der beiden Schichten 19A, 19B ist wiederum eine Bohrung für den Strahlungsleiter 21 vorgesehen. In zumindest einer der beiden Schichten 19A, 19B ist eine Aufnahme für das zumindest eine Halbleiterelement 7 oder eine Bohrung, die zumindest einen Teil der Kammer 22 bildet, angeordnet.

Die Figur 8 zeigt eine Beleuchtungsvorrichtung 3C, deren Aufbau im Wesentlichen den im Vorstehenden beschriebenen Beleuchtungsvorrichtung 3, 3A, 3B entspricht, so dass zur Vermeidung von Wiederholungen gleiche Bauteile wiederum mit den gleichen Bezugszeichen versehen sind. Das Strahlungsabgabeende 9 der Beleuchtungsvorrichtung 3C weist jedoch nur eine, insbesondere für sichtbares Licht, transparente Licht- oder Strahlungsabgabefläche 8 auf, die im Wesentlichen ringförmig ausgebildet ist oder sich um die Öffnung 5 erstreckt. Das Strahlungsabgabeende 9 oder die Strahlungsabgabefläche 8 ist vorzugsweise durch eine Kunststoff-, Kunstharz- oder Glasschicht 20 gebildet. Die Schicht 20 reicht entweder bis zu dem zumindest einen Halbleiterelement 7, welches somit direkt elektromagnetische Strahlung, insbesondere sichtbares Licht, an die Schicht 20 abgibt, oder zwischen der Schicht 20 und dem zumindest einen Halbleiterelement 7 ist zumindest ein optischer Strahlungsleiter, insbesondere ein Strahlungsleiter 21, wie er im Vorstehenden beschrieben wurde, vorgesehen, der die Strahlung zur Schicht 20 leitet.

In der Schicht 20 sind des Weiteren die Öffnungen 11 zur Abgabe von Fluid vorgesehen. Die Öffnungen 11 sind somit durch Strahlung abgebende oder, insbesondere für sichtbares Licht, transparente Abschnitte der Strahlungsabgabefläche 8 voneinander getrennt.

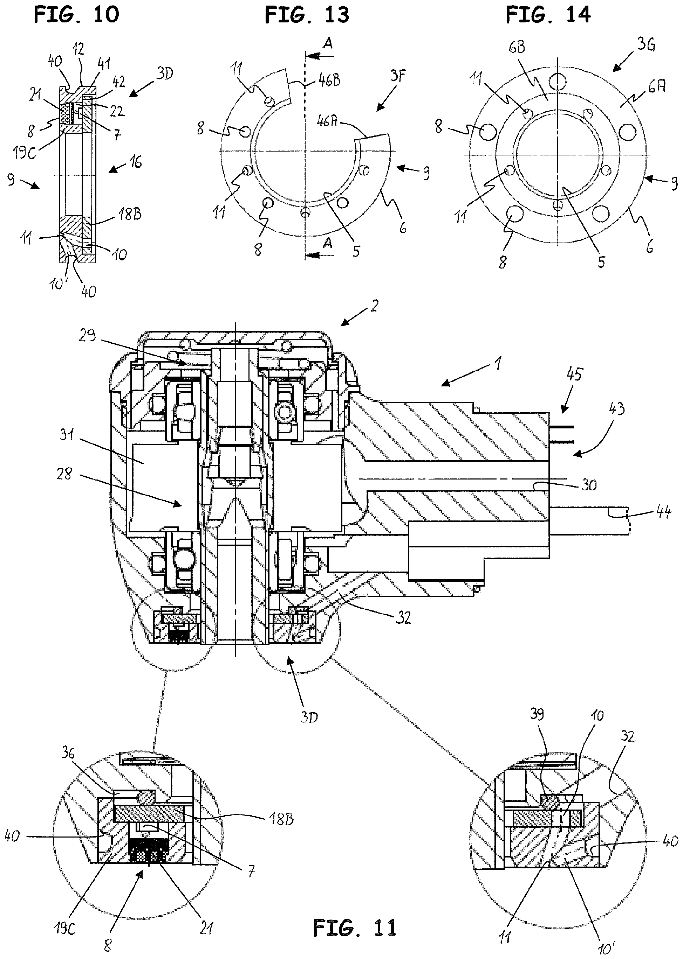

Die Beleuchtungsvorrichtung 3D der Figur 10 unterscheidet sich von den im Vorstehenden beschriebenen Beleuchtungsvorrichtungen insbesondere darin, dass an ihrer Mantelfläche 12 ein ringförmiger Kanal 40 vorgesehen ist. Der Ringkanal 40 ist zur Aufnahme eines Fluids, insbesondere von Wasser, ausgebildet und, vorzugsweise über Leitungen und / oder Kanäle im Instrument 1 mit einer Fluidquelle verbindbar oder verbunden. Der Ringkanal 40 ist mit der Fluidabgabeöffnung 11 und / oder mit der Mischkammer 13 verbunden, insbesondere über den Fluidkanal 10'. Ein derartiger Ringkanal 40 kann auch bei den Beleuchtungsvorrichtungen 3, 3A, 3B oder 3C vorgesehen sein.

Die Beleuchtungsvorrichtung 3D umfasst des Weiteren zwei Schichten, wobei bevorzugt die Schicht 18B aus Keramik und die Schicht 19C aus Metall besteht. In der Schicht 19C ist der Ringkanal 40 vorgesehen. Die Schicht 19C weist einen Flansch 41 und / oder eine Schulter 42 zur Verbindung oder Lagerung der Schicht 18B auf. Die Schulter 42 und der Ringkanal 40 sind vorzugsweise an unterschiedlichen oder gegenüber liegenden Seiten der Beleuchtungsvorrichtung 3D angeordnet. In zumindest einer Bohrung der Schicht 19C ist zumindest ein optischer Strahlungsleiter 21 aufgenommen.

Die Figur 9 zeigt eine Schnittdarstellung durch einen Instrumentenkopf 2 eines Instruments 1 mit einer Beleuchtungsvorrichtung 3A gemäß der Figur 6 (selbstverständlich sind jedoch in entsprechender Weise die Beleuchtungsvorrichtungen 3, 3B, 3C, 3D in den Instrumentenkopf 2 implementierbar). Die Beleuchtungsvorrichtung 3A ist in einer, insbesondere kreis- oder ringförmige, Aufnahme 36 oder einem Rücksprung des Instrumentenkopfs 2, vorzugsweise lösbar, angeordnet. Als Befestigungsmittel für die Beleuchtungsvorrichtung 3A ist eine Gewindehülse 37 vorgesehen, die mit einem Gewindebauteil des Instruments 1 verbindbar ist und die Beleuchtungsvorrichtung 3A in der Aufnahme 36 fixiert, zum Beispiel klemmt. Dazu sind an der Gewindehülse 37 und an der Beleuchtungsvorrichtung 3A Kontaktflächen 38A, 38B vorgesehen. Vorzugsweise ist die Gewindehülse 37 derart bemessen, dass sie in der Öffnung 5 der Beleuchtungsvorrichtung 3A aufnehmbar ist. Vorzugsweise ist die Gewindehülse 37 derart bemessen, dass zumindest ein Teil der Werkzeughaltevorrichtung 28 in der Innenbohrung der Gewindehülse 37 aufnehmbar ist.

Die Aufnahme 36 dient auch als Verbindungsstück zwischen der Medien- oder Fluidleitung 32 und dem zumindest einen Fluidkanal 10 oder als Leitungsstück, welches Fluid von der Medien- oder Fluidleitung 32 an den zumindest einen Fluidkanal 10 leitet. Insbesondere ist die Aufnahme 36 zumindest als Teil eines Kanals ausgebildet, zum Beispiel ringförmig oder als Ringkanal, der ein Fluid an mehrere Fluidkanäle 10, 10' abgibt. Vorzugsweise ist in der Aufnahme 36 ein Dichtmittel 39, zum Beispiel eine O-Ring, vorgesehen.

Schließlich ist in der Figur 9 eine Steuer- und / oder Versorgungsvorrichtung 23 dargestellt, die durch die Anschlussvorrichtung 24 mit dem Instrument 1 verbunden ist. Die Steuer- und / oder Versorgungsvorrichtung 23 versorgt das Instrument 1 über einen Versorgungsschlauch mit Medien, insbesondere Fluiden, elektrischer Energie und / oder Steuersignalen und / oder steuert oder überwacht den Betrieb des Instruments 1. Die Steuer- und / oder Versorgungsvorrichtung 23 stellt somit bevorzugt auch die elektrische Energie für das zumindest eine Halbleiterelement 7 und das zumindest eine Fluid zur Verfügung, welches über die Beleuchtungsvorrichtung 3A abgebbar ist.

Die Steuer- und / oder Versorgungsvorrichtung 23 ist vorzugsweise ausgebildet, eine alternierende Abgabe der elektromagnetischen Strahlung und des Fluids vom Licht- oder Strahlungsabgabeende 9 der Beleuchtungsvorrichtung 3A zu steuern. Dies erfolgt zum Beispiel mittels entsprechender elektrischer Steuersignale an das Halbleiterelement 7, insbesondere durch eine gepulste Versorgung des Halbleiterelements 7 mit elektrischer Energie, und durch das Öffnen und Schließen eines Steuerelements, zum Beispiel eines Ventils, in einer mit der Öffnung 11 zur Fluidabgabe verbundenen Medien- oder Fluidleitung 32.

Die Figur 11 zeigt eine Schnittdarstellung durch einen Instrumentenkopf 2 eines Instruments 1 mit einer Beleuchtungsvorrichtung 3D gemäß der Figur 10 (selbstverständlich sind jedoch in entsprechender Weise die Beleuchtungsvorrichtungen 3, 3A, 3B, 3C in den Instrumentenkopf 2 implementierbar). In den Figuren 9 und 11 dargestellte, gleiche Bauteile tragen dieselben Bezugszeichen.

Die Beleuchtungsvorrichtung 3D ist unlösbar mit dem Instrumentenkopf 2 verbunden, vorzugsweise durch Verpressen der Beleuchtungsvorrichtung 3D mit dem Instrumentenkopf 2, insbesondere durch Einpressen der Beleuchtungsvorrichtung 3D in die Aufnahme 36, oder durch eine kraftschlüssige Verbindung oder eine Reibverbindung zwischen der Beleuchtungsvorrichtung 3D und dem Instrumentenkopf 2, insbesondere der Aufnahme 36.

Um gegebenenfalls die Beleuchtungsvorrichtung 3D austauschen zu können, ist in einem bevorzugten Ausführungsbeispiel vorgesehen, dass der Instrumentenkopf 2 gemeinsam mit der Beleuchtungsvorrichtung 3D von dem Instrument 1, insbesondere von dem Griffteil 25, lösbar ist. Dazu weist der Instrumentenkopf 2 ein Kupplungselement 43 auf, das mit einem entsprechenden Kupplungselement des Griffteils 25 verbindbar ist und eine Kupplungsvorrichtung bildet. An dem Kupplungselement 43 ist zumindest eine Fluid- oder Medienleitung 30, 32, 44 vorgesehen, insbesondere für Luft oder Wasser, die mit zumindest einer entsprechenden Leitung im Griffteil 25 oder an dessen Kupplungselement, vorzugsweise mittels Stecken, verbindbar ist. An dem Kupplungselement 43 ist des Weiteren eine elektrische Verbindungsvorrichtung 45 vorgesehen, insbesondere zwei elektrische Kontakte, die mit einer entsprechenden elektrischen Verbindungsvorrichtung an dem Kupplungselement des Griffteils 25 verbindbar ist. Über diese elektrische Verbindungsvorrichtung ist die Beleuchtungsvorrichtung 3D, insbesondere das zumindest eine Halbleiterelement 7, mit einer elektrischen Energiequelle verbindbar und mit elektrischer Energie versorgbar.

Die in den Figuren 9 und 11 dargestellten Instrumente 1 sind als druckluftbetriebene Instrumente mit einem Laufrad 31 ausgebildet. Selbstverständlich ist es in entsprechender Weise möglich, eine Beleuchtungsvorrichtung 3 - 3G in ein Instrument mit einem mechanischen Antrieb des Werkzeugs 4 zu implementieren, wobei der mechanische Antrieb zumindest eine Welle umfasst, die eine Antriebsbewegung auf die Werkzeughaltevorrichtung 28 und / oder das Werkzeug 4 überträgt.

Die Figur 12 zeigt eine Beleuchtungsvorrichtung 3E, deren Körper 6 zumindest teilweise oder vollständig aus Vergussmaterial oder Spritzgussmaterial hergestellt ist, zum Beispiel aus Kunstharz, insbesondere aus Epoxidharz, oder aus einem thermoplastischen Kunststoff, zum Beispiel aus statistischen Propylen-Copolymeren (PPR), Polycarbonat (PC), Polymethylenpenten (PMP), Cyclooleofin-Copolymeren (COC) oder Polyphenylsulfon (PPSU). Vorzugsweise sind das zumindest eine Halbleiterelement 7 und / oder der zumindest eine Fluidkanal 10, 10', 40 und / oder der zumindest eine elektrische Leiter 17 und / oder die Kammer 22 und / oder der optische Strahlungsleiter 21 in das Vergussmaterial oder Spritzgussmaterial eingebettet oder von diesem zumindest teilweise umgeben. Im Übrigen entspricht die Beleuchtungsvorrichtung 3E im Wesentlichen den im Vorstehenden beschriebenen Beleuchtungsvorrichtungen 3 - 3D.

Bei den in den Figuren 1 - 12 dargestellten Beleuchtungsvorrichtungen 3 - 3E ist der Körper 6 jeweils als geschlossener Ring oder Hohlzylinder ausgebildet. Selbstverständlich besteht jedoch auch die Möglichkeit, die Beleuchtungsvorrichtung mit zwei freien Enden oder Endflächen 46A, 46B auszubilden, insbesondere gebogen oder bogenförmig oder als Kreisbogen, so wie dies beispielhaft anhand der Beleuchtungsvorrichtung 3F in der Figur 13 dargestellt ist. Zwischen den freien Enden 46A, 46B ist ein Freiraum oder eine Öffnung vorgesehen. Im Übrigen entspricht die Beleuchtungsvorrichtung 3F im Wesentlichen wiederum den im Vorstehenden beschriebenen Beleuchtungsvorrichtungen 3 - 3E. Selbstverständlich besteht auch die Möglichkeit, die Beleuchtungsvorrichtung eckig geschlossen oder eckig mit freien Enden (gewinkelt) auszubilden.

Die Figur 14 zeigt eine Beleuchtungsvorrichtung 3G, deren Körper 6 aus zwei Teilen 6A, 6B besteht, wobei an einem Teil 6A das Strahlungsabgabeende 9 und / oder die zumindest eine Strahlungsabgabefläche 8 und an dem anderen Teil 6B die zumindest eine Öffnung 11 zur Abgabe von Fluid vorgesehen ist. Vorzugsweise sind an einem der beiden Teile 6A, 6B, insbesondere an jenem Teil 6A mit dem Strahlungsabgabeende 9 und / oder der zumindest einen Strahlungsabgabefläche 8, das zumindest eine Halbleiterelement 7 und / oder der zumindest eine elektrische Leiter 17 und / oder die Kammer 22 und / oder der optische Strahlungsleiter 21 vorgesehen. Alternativ oder zusätzlich sind vorzugsweise an einem der beiden Teile 6A, 6B, insbesondere an jenem Teil 6B mit der zumindest einen Öffnung 11 zur Abgabe von Fluid der zumindest eine Fluidkanal 10, 10', 40 vorgesehen. Vorzugsweise umgibt ein Teil 6A, 6B den anderen Teil 6A, 6B. Vorzugsweise sind die beiden Teile 6A, 6B konzentrisch angeordnet. Vorzugsweise sind die beiden Teile 6A, 6B unlösbar miteinander verbunden. Vorzugsweise sind die beiden Teile 6A, 6B aus gleichem oder unterschiedlichem Material hergestellt. Im Übrigen entspricht die Beleuchtungsvorrichtung 3G im Wesentlichen den im Vorstehenden beschriebenen Beleuchtungsvorrichtungen 3 - 3F.

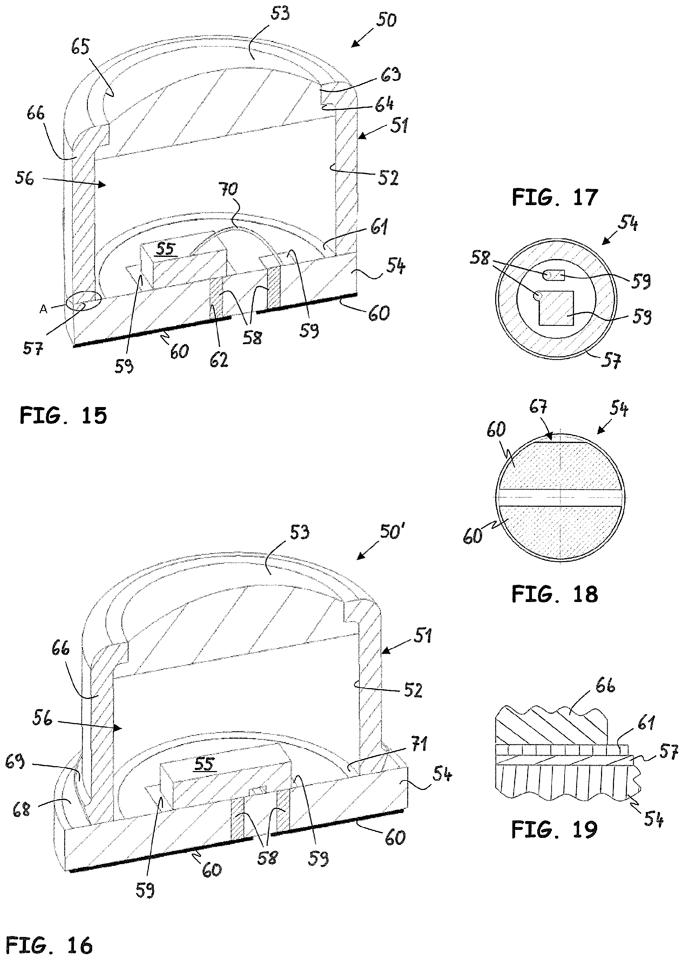

Die Figuren 15 und 16 zeigen zwei Ausführungsbeispiele von Beleuchtungsvorrichtungen 50, 50'. Aufgrund ihres sehr ähnlichen Aufbaus gilt die folgende Beschreibung für beide Figuren 15 und 16 und es sind gleiche oder ähnliche Bauteile mit denselben Bezugszeichen versehen. Die Beleuchtungsvorrichtungen 50, 50' sind vorzugsweise zum Befestigen an einem Instrument oder Instrumentenkopf vorgesehen, insbesondere an oder in einem Instrument 1 oder Instrumentenkopf 2 wie in den Figuren 1, 9 und 11 dargestellt und beschrieben. Demgemäß sind alle in Verbindung mit den Figuren 1, 9 oder 11 beschriebenen Merkmal der Instrumente 1 oder Instrumentenköpfe 2 auch auf ein Instrument oder einen Instrumentenkopf mit einer Beleuchtungsvorrichtung 50, 50' übertragbar.

Die Beleuchtungsvorrichtung 50, 50' umfasst eine hohle, metallische Hülse 51, ein transparentes Fenster 53 zur Abgabe elektromagnetischer Strahlung, insbesondere von sichtbarem Licht, und einen Sockel 54. Auf dem Sockel 54 ist ein optisches Halbleiterelement 55 vorgesehen, welches ausgebildet ist, elektromagnetische Strahlung, insbesondere sichtbares Licht, zu emittieren.

Die hohle, metallische Hülse 51 ist insbesondere als zylindrische Hülse oder Hohlzylinder mit einer Durchbohrung 52 ausgebildet. Die Durchbohrung 52 bildet an einem Ende der Hülse 51 eine runde oder kreisrunde Öffnung 65. An einem Ende der Hülse 51 oder der Durchbohrung 52, in der Öffnung 65 ist das transparente Fenster 53 vorgesehen, wobei komplementäre Schulter oder Vorsprünge oder Flansche 63, 64 an der Hülse 51 und dem Fenster 53 vorgesehen sind, so dass das Fenster 53 an der Hülse 51 gelagert ist. Das transparente Fenster 53 ist vorzugsweise in die hohle, metallische Hülse 51 eingeschmolzen. Das transparente Fenster 53 umfasst bevorzugt eine konvexe, zum Beispiel linsenförmige, Form zur Bündelung der von dem optischen Halbleiterelement 55 emittierten elektromagnetischen Strahlung.

An dem der Öffnung 65 gegenüber liegenden Ende der Hülse 51 bildet die Durchbohrung 52 eine weitere, insbesondere runde oder kreisrunde, Öffnung. An oder in dieser Öffnung ist der Sockel 54 vorgesehen, der aus keramischem Material oder aus glaskeramischem Material oder aus Glas gebebildet ist. Der Sockel 54 ist rund oder flach oder im Wesentlichen plattenförmig ausgebildet und weist einen Außenmantel, eine Oberseite und eine im Wesentlichen parallel zur Oberseite angeordnete Unterseite auf. Das transparente Fenster 53 und der Sockel 54 verschließen die Durchbohrung 52 oder die Öffnungen 65 der hohlen, metallischen Hülse 51 derart, dass eine Kammer 56 gebildet ist, in welcher das zumindest eine optische Halbleiterelement 55 aufgenommen ist. Das optische Halbleiterelement 55 ist auf dem Sockel 54 befestigt, insbesondere auf der in das Innere der Kammer 56 weisenden Oberseite des Sockels 54.

Der Sockel 54 weist mehrere elektrische Kontakte zur elektrischen Verbindung des optischen Halbleiterelements 55 mit einer elektrischen Energiequelle auf: Zwei elektrische, vorzugsweise stiftförmige oder leitungsartige, Kontakte 58 sind in Bohrungen 62 des Sockels 54 aufgenommen oder durchsetzen den Sockel 55, insbesondere derart, dass die Kontakte 58 an unterschiedlichen Oberflächen des Sockels 54 enden, zum Beispiel an dessen Oberseite und Unterseite. Jeweils zwei weitere elektrische Kontakte oder Flächenkontakte 59, 60 sind an Oberflächen des Sockels 54 vorgesehen, insbesondere an dessen Oberseite und an dessen Unterseite. Das zumindest eine optische Halbleiterelement 55 ist mit einem oder mehreren der Kontakte 58, 59, 60 direkt oder indirekt verbunden, vorzugsweise ist auf zumindest einem der Flächenkontakte 59 das zumindest eine optische Halbleiterelement 55 angeordnet. Die elektrischen Kontakte 58, 59, 60 sind elektrisch miteinander verbunden und sind Teil eines mit einer elektrischen Energiequelle verbundenen oder verbindbaren Schalt- oder Stromkreises.

Wie insbesondere in Verbindung mit der Figur 17 zu erkennen ist, sind an der Oberseite des Sockels 54 zwei Flächenkontakte 59, die zum Beispiel einen elektrischen Plus- und Minuspol bilden, und das zumindest eine optische Halbleiterelement 55 angeordnet. In der Figur 17 sind auch die oberseitigen Enden der beiden elektrischen Kontakte 58 angedeutet, die mit jeweils einem der Flächenkontakte 59 elektrisch verbunden sind. An der Unterseite des Sockels 54 sind die beiden Flächenkontakte 60 vorgesehen, die zum Beispiel einen elektrischen Plus- und Minuspol bilden, siehe auch Figur 18. Die Unterseite des Sockels 54 mit den beiden Flächenkontakten 60 bildet vorzugsweise auch eine Außenseite der Beleuchtungsvorrichtung 50, 50'. Zum Zwecke einer korrekten Montage, insbesondere einer korrekten elektrischen Kontaktierung des zumindest einen optischen Halbleiterelements 55, kann an zumindest einem elektrischen Kontakt 58, 59, 60 eine Markierung 67 vorgesehen sein, zum Beispiel kann ein Kontakt 58, 59, 60 unterschiedlich geformt sein oder es ist ein alphanumerisches Zeichen vorgesehen.

Die elektrischen Kontakte 58, 59, 60 umfassen ein Metall und Glas aufweisendes Material, insbesondere ein Metall- und Glaspartikel aufweisendes Gemisch, dessen Partikel durch Erwärmen, insbesondere Sintern, miteinander und mit dem Sockel 54 verbunden oder verschmolzen sind. Das Metall und Glas aufweisende Material ist / wird, vorzugsweise vor dem Erwärmen oder Sintern, auf dem Sockel 54 aufgebracht und / oder in eine Bohrung 62 des Sockels 54 eingebracht. Insbesondere die Flächenkontakte 59, 60 weisen zumindest eine weitere elektrisch leitende Schicht auf, zum Beispiel eine Gold aufweisende Schicht oder Legierung. Diese weitere elektrisch leitende Schicht ist auf das Metall und Glas aufweisende Material aufgebracht, insbesondere nach dem Erwärmen oder Sintern des Sockels 54 und / oder des Metall und Glas aufweisenden Materials.

Im Folgenden werden die Unterschiede der in den Figuren 15 und 16 dargestellten Beleuchtungsvorrichtungen 50, 50' beschrieben:
Bei der Beleuchtungsvorrichtungen 50 der Figur 15 umfasst die Verbindung zwischen dem Sockel 54 und der hohlen, metallischen Hülse 51 oder das den Sockel 54 und die Hülse 51 verbindende Material ein Metall und Glas aufweisendes Material 57, insbesondere ein Metall- und Glaspartikel aufweisendes Gemisch, dessen Partikel durch Erwärmen, insbesondere durch Sintern des Sockels 54, miteinander und mit dem Sockel 54 und / oder mit der Hülse 51 verbunden oder verschmolzen sind. Vorzugsweise ist das Metall und Glas aufweisende Material 57 zur Verbindung des Sockels 54 mit der Hülse 51 und das Metall und Glas aufweisende Material der elektrischen Kontakte 58, 59, 60 dasselbe und / oder wird innerhalb eines Verfahrensschritts gemeinsam auf den Sockel 54 und gegebenenfalls auf die Hülse 51 aufgebracht, vorzugsweise vor dem Erwärmen oder Sintern.

Die Verbindung zwischen dem Sockel 54 und der hohlen, metallischen Hülse 51 oder das den Sockel 54 und die Hülse 51 verbindende Material umfasst des Weiteren eine Metalllegierung 61, zum Beispiel ein Metalllot. Die Metalllegierung ist / wird auf das als Träger oder Haftvermittler dienende Metall und Glas aufweisende Material 57 aufgebracht, vorzugsweise nach dem Erwärmen oder Sintern des Metall und Glas aufweisenden Materials 57. Die Verbindung zwischen dem Sockel 54 und der hohlen, metallischen Hülse 51 umfasst somit mehrere Materialien oder Werkstoffe oder Komponenten, die insbesondere schichtweise ausgebildet oder nacheinander aufgebracht sind (siehe auch Figur 19).

Bei der Beleuchtungsvorrichtungen 50' der Figur 16 umfasst die Verbindung zwischen dem Sockel 54 und der hohlen, metallischen Hülse 51 oder das den Sockel 54 und die Hülse 51 verbindende Material ein Verguß- oder Klebematerial 71, zum Beispiel ein Silikon- oder Epoxidharz oder einen Silikon- oder Epoxidkleber. Das Verguß- oder Klebematerial 71 ist gemäß einem Ausführungsbeispiel zwischen dem Kontaktbereich des Sockels 54 und der Hülse 51 oder an einem Verbindungsbereich, dessen Breite im Wesentlichen der Wandstärke der Wand 66 entspricht, vorgesehen. Alternativ oder zusätzlich ist das Verguß- oder Klebematerial 71 auch an der Außenseite des Sockels 54, zum Beispiel an einem Flansch 68, und / oder an der Außenseite der Hülse 51, zum Beispiel an einem Flansch 69, vorgesehen. Alternativ oder zusätzlich ist das Verguß- oder Klebematerial 71 im Inneren der Hülse 51 und / oder in der Kammer 56 vorgesehen. Besonders bevorzugt bedeckt oder umschließt das Verguß- oder Klebematerial 71 zumindest eine Seite oder Fläche des optischen Halbleiterelements 55, wahlweise mehrere Seiten oder Flächen das optischen Halbleiterelements 55 einschließlich der dem transparenten Fenster 53 zugewandten Seite oder Fläche, und bildet somit insbesondere eine Schutzschicht für das optische Halbleiterelement 55.

Selbstverständlich ist auch eine Kombination der beiden im Vorstehenden beschriebenen Arten der Verbindung zwischen dem Sockel 54 und der hohlen, metallischen Hülse 51 möglich, also eine Verbindung die ein Metall und Glas aufweisendes Material 57 und ein Verguß- oder Klebematerial 71 und vorzugsweise auch eine Metalllegierung 61 aufweist. Bei diesem Ausführungsbeispiel ist insbesondere das Metall und Glas aufweisende Material 57 und gegebenenfalls die Metalllegierung 61 zwischen dem Sockel 54 und der Hülse 51 (so wie in der Figur 15 und 19 dargestellt) oder an dem oben genannten Verbindungsbereich und das Verguß- oder Klebematerial 71 in der Kammer 56 und / oder an der Außenseite der Beleuchtungsvorrichtung vorgesehen.

Bei der Beleuchtungsvorrichtungen 50 der Figur 15 weisen die Hülse 51 und der Sockel 54 im Wesentlichen den gleichen Außendurchmesser auf, so dass der Außendurchmesser der Beleuchtungsvorrichtungen 50 in etwa dem Außendurchmesser der Hülse 51 und des Sockels 54 entspricht. Zur Herstellung einer dichten, festen Verbindung zwischen dem Sockel 54 und der Hülse 51 ist die Wandstärke der Wand 66 der Hülse 51 im Wesentlichen ausreichend. In anderen Worten ist die Verbindung zwischen dem Sockel 54 und der Hülse 51 durch einen Verbindungsbereich definiert, dessen Breite im Wesentlichen der Wandstärke der Wand 66 entspricht. Im Gegensatz dazu ist der Durchmesser des Sockels 54 der Beleuchtungsvorrichtungen 50' der Figur 16 breiter als der Durchmesser der Hülse 51, so dass ein Flansch 68 gebildet ist, der zum Beispiel zur Befestigung der Beleuchtungsvorrichtungen 50 an dem Instrument 1 dient. Gegebenenfalls kann auch die Hülse 51 an ihrem Ende, an dem sie mit dem Sockel 54 verbunden ist, eine Verbreiterung oder einen Flansch 69 aufweisen, so dass der Verbindungsbereich zwischen dem Sockel 54 und der Hülse 51 breiter ist als die Wandstärke der Wand 66 der Hülse 51. Selbstverständlich kann ein Flansch 68, 69 auch bei der Beleuchtungsvorrichtungen 50 der Figur 15 vorgesehen sein oder die Beleuchtungsvorrichtungen 50' der Figur 16 flanschlos, entsprechend der Figur 15 ausgebildet sein.

Ein weiterer Unterschied zwischen den Beleuchtungsvorrichtungen 50 und 50' besteht in der Anordnung und / oder Ausbildung des zumindest einen optischen Halbleiterelements 55. Bei dem optischen Halbleiterelement 55 der Figur 15 sind der elektrische Pluspol und der elektrische Minuspol an unterschiedlichen, insbesondere entgegengesetzten, Seiten des optischen Halbleiterelements 55 angeordnet. Zur Verbindung des Pluspols und Minuspols mit den elektrischen Kontakten 58, 59, 60 ist vorgesehen, das optische Halbleiterelement 55 mit einem der beiden Pole direkt auf einem ersten Flächenkontakt 59 auf der Oberseite des Sockels 54 anzuordnen und den anderen der beiden Pole mit einem zweiten Flächenkontakt 60 auf der Oberseite des Sockels 54 über einen elektrischen Leiter 70, zum Beispiel über einen Draht, insbesondere über einen Golddraht, zu verbinden. Das optische Halbleiterelement 55 der Beleuchtungsvorrichtungen 50' der Figur 16 weist beide elektrischen Pole an der gleichen Seite auf (so genannter Flip-Chip), so dass das optische Halbleiterelement 55 mit beiden Polen direkt auf den beiden Flächenkontakten 59 auf der Oberseite des Sockels 54 anordenbar ist. Selbstverständlich ist es auch möglich, einen Flip-Chip in der Beleuchtungsvorrichtungen 50 der Figur 15 oder ein optisches Halbleiterelement 55 mit einem elektrischen Leiter 70 in der Beleuchtungsvorrichtungen 50' der Figur 16 vorzusehen.

Die Erfindung ist nicht auf die beschriebenen Ausführungsbeispiele beschränkt, sondern ist durch die angehängten Ansprüche definiert. Es kann gemäß einem Ausführungsbeispiel der Körper einer Beleuchtungsvorrichtung, in dem zumindest ein Fluidkanal und / oder zumindest eine Öffnung zur Abgabe von Fluid vorgesehen ist, so wie in den Figuren 1 - 14 dargestellt und in der zugehörigen oder darauf Bezug nehmenden Beschreibung offenbart, einen Sockel aus keramischem Material oder aus glaskeramischem Material oder aus Glas aufweisen, welcher mit einer hohlen, metallischen Hülse durch ein Metall und Glas aufweisendes Material verbunden ist, so wie dies in den Figuren 15 - 18 dargestellt und in der zugehörigen oder darauf Bezug nehmenden Beschreibung offenbart ist.

## Patentansprüche

1. Beleuchtungsvorrichtung (3 - 3G) für einen Instrumentenkopf (2) eines medizinischen, insbesondere dentalen, Instruments (1), wobei an dem Instrumentenkopf (2) ein Werkzeug (4) zum Einwirken auf eine Behandlungsstelle vorgesehen ist und wobei die Beleuchtungsvorrichtung (3 - 3G) umfasst: Eine Aufnahme oder Öffnung (5), in welcher das Werkzeug (4) aufnehmbar ist, einen die Aufnahme oder Öffnung (5) umgebenden Körper (6), welcher ein Strahlungsabgabeende (9) mit zumindest einer optisch leitenden, insbesondere transparenten, Strahlungsabgabefläche (8), ein Instrumentenanschlussende (16) und eine sich zwischen dem Strahlungsabgabeende (9) und dem Instrumentenanschlussende (16) erstreckende Mantelfläche (12) aufweist, zumindest ein am Körper (6) vorgesehenes Halbleiterelement (7), das zur Emission von elektromagnetischer Strahlung ausgebildet ist, so dass elektromagnetische Strahlung von der zumindest einen optisch leitenden Strahlungsabgabefläche (8) des Strahlungsabgabeendes (9) abgebbar ist und zumindest eine am Strahlungsabgabeende (9) des Körpers (6) der Beleuchtungsvorrichtung (3 - 3G) vorgesehene Öffnung (11) zur Abgabe von Fluid, so dass vom Strahlungsabgabeende (9) des Körpers (6) zusätzlich zumindest ein Fluid oder ein Fluidgemisch abgebbar ist, und einen an der Mantelfläche (12) vorgesehenen ringförmigen Kanal (40) zur Aufnahme eines Fluids, insbesondere von Wasser, der mit der Fluidabgabeöffnung (11) verbunden ist.

2. Beleuchtungsvorrichtung (3 - 3G) nach Anspruch 1, **gekennzeichnet durch** zumindest einen in der Beleuchtungsvorrichtung (3 - 3G), insbesondere innerhalb des in sich geschlossenen Außenumfangs der Mantelfläche (12) des Körpers (6), vorgesehenen Fluidkanal (10, 10'), der die zumindest eine am Strahlungsabgabeende (9) der Beleuchtungsvorrichtung (3 - 3G) vorgesehene Öffnung (11) zur Abgabe von Fluid mit dem ringförmigen Kanal (40) verbindet.

3. Beleuchtungsvorrichtung (3 - 3G) nach Anspruch 1 oder 2, **gekennzeichnet durch**
mehrere in der Beleuchtungsvorrichtung (3 - 3G), insbesondere innerhalb des in sich geschlossenen Außenumfangs der Mantelfläche (12) des Körpers (6), vorgesehene Fluidkanäle (10, 10') die in eine gemeinsame Fluidmischkammer (13) in der Beleuchtungsvorrichtung (3 - 3G) münden, wobei die Fluidmischkammer (13) mit der zumindest einen Öffnung (11) zur Abgabe von Fluid verbunden ist.

4. Beleuchtungsvorrichtung (3 - 3G) nach einem der Ansprüche 1 - 3, **gekennzeichnet durch**
mehrere in der Beleuchtungsvorrichtung (3 - 3G), insbesondere innerhalb des in sich geschlossenen Außenumfangs der Mantelfläche (12) des Körpers (6), vorgesehene Fluidkanäle (10, 10', 40), wobei zumindest ein Fluidkanal (10') eine Öffnung (14) zur Verbindung mit einer Fluidquelle an der Mantelfläche (12) des Körpers (6) der Beleuchtungsvorrichtung (3 - 3G) und zumindest ein Fluidkanal (10) eine Öffnung (15) zur Verbindung mit einer Fluidquelle am Instrumentenanschlussende (16) des Körpers (6) der Beleuchtungsvorrichtung (3 - 3G) aufweist.

5. Beleuchtungsvorrichtung (3 - 3G) nach einem der Ansprüche 1 - 4, **gekennzeichnet durch** mehrere optisch leitende Strahlungsabgabeflächen (8) und mehrere Öffnungen (11) zur Abgabe von Fluid, wobei zwischen zwei Öffnungen (11) zur Abgabe von Fluid zumindest eine Strahlungsabgabefläche (8) angeordnet ist.

6. Beleuchtungsvorrichtung (3 - 3G) nach einem der Ansprüche 1 - 5, **dadurch gekennzeichnet, dass**
mehrere elektrisch seriell geschaltete, mittels eines elektrischen Leiters (17) verbundene Halbleiterelemente (7) vorgesehen sind, wobei der elektrische Leiter (17) beabstandet von dem zumindest einen in der Beleuchtungsvorrichtung (3 - 3G) vorgesehenen Fluidkanal (10, 10', 40) angeordnet ist.

7. Beleuchtungsvorrichtung (3 - 3G) nach einem der Ansprüche 1 - 6, **dadurch gekennzeichnet, dass**
die Beleuchtungsvorrichtung (3 - 3G) aus mehreren Schichten (18, 19), insbesondere aus mehreren Schichten unterschiedlicher Materialien, aufgebaut ist.

8. Beleuchtungsvorrichtung (3 - 3G) nach Anspruch 7, **dadurch gekennzeichnet, dass**
zumindest eine der Schichten metallisches Material (19, 19A, 19B, 19C) und / oder Kunststoff und / oder Glas (20) umfasst, insbesondere jene Schicht, die das Strahlungsabgabeende (9) der Beleuchtungsvorrichtung (3 - 3G) bildet.

9. Beleuchtungsvorrichtung (3 - 3G) nach Anspruch 7 oder 8, **dadurch gekennzeichnet, dass**
die Beleuchtungsvorrichtung (3 - 3G) zwei Schichten (18B, 19C) umfasst, wobei eine erste Schicht (19C), in welcher der ringförmige Kanal (40) vorgesehen ist, aus Metall besteht, wobei die erste Schicht (19C) einen Flansch (41) und / oder eine Schulter (42) zur Verbindung oder Lagerung der zweiten Schicht (18B) aufweist und wobei in zumindest einer Bohrung der ersten Schicht (19C) zumindest ein optischer Strahlungsleiter (21) aufgenommen ist.

10. Beleuchtungsvorrichtung (3 - 3G) nach einem der Ansprüche 1 - 9, **dadurch gekennzeichnet, dass**
mehrere Halbleiterelemente (7) vorgesehen sind, wobei jedem Halbleiterelement (7) ein eigener optischer Strahlungsleiter (21) zugeordnet ist, der derart angeordnet ist, dass er die von den Halbleiterelementen (7) emittierte elektromagnetische Strahlung in Richtung des Strahlungsabgabeendes (9) der Beleuchtungsvorrichtung (3 - 3G) leitet.

11. Beleuchtungsvorrichtung (3 - 3G) nach einem der Ansprüche 1 - 8, **dadurch gekennzeichnet**, ass das
Strahlungsabgabeende (9) der Beleuchtungsvorrichtung (3 - 3G) nur eine, insbesondere für sichtbares Licht, transparente Licht- oder Strahlungsabgabefläche (8) aufweist, die im Wesentlichen ringförmig ausgebildet ist oder sich um die Öffnung (5), in welcher das Werkzeug (4) aufnehmbar ist, erstreckt.

12. Instrumentenkopf (2) eines medizinischen, insbesondere dentalen, Instruments (1), **gekennzeichnet durch**
eine Beleuchtungsvorrichtung (3 - 3G) gemäß einem der Ansprüche 1 - 11.

13. Instrumentenkopf (2) eines medizinischen, insbesondere dentalen, Instruments (1) nach Anspruch 12, **gekennzeichnet durch**
eine Aufnahme (36), zum Beispiel einen Rücksprung, an dem Instrumentenkopf (2), in der die Beleuchtungsvorrichtung (3 - 3G), insbesondere lösbar, aufnehmbar ist, wobei bevorzugt die Aufnahme (36) ausgebildet ist, Fluid an die Beleuchtungsvorrichtung (3 - 3G) zu übertragen.

14. Instrumentenkopf (2) eines medizinischen, insbesondere dentalen, Instruments (1) nach Anspruch 13, **dadurch gekennzeichnet, dass**
zumindest ein Teil der Aufnahme (36) als Fluidkanal dient, welcher der Beleuchtungsvorrichtung (3 - 3G) ein Fluid zuführt oder dass zumindest ein Teil der Aufnahme (36) gemeinsam mit der Beleuchtungsvorrichtung (3 - 3G), zum Beispiel einer Ausnehmung der Beleuchtungsvorrichtung (3 - 3G), zumindest einen Abschnitt des Fluidkanals der Beleuchtungsvorrichtung (3 - 3G) bildet, oder dass die Aufnahme (36) zumindest als Teil eines Kanals ausgebildet ist, zum Beispiel ringförmig oder als Ringkanal, der ein Fluid an mehrere Fluidkanäle (10, 10') abgibt.

15. Instrumentenkopf (2) eines medizinischen, insbesondere dentalen, Instruments (1), **gekennzeichnet durch**
eine Beleuchtungsvorrichtung (3 - 3G) für einen Instrumentenkopf (2) eines medizinischen, insbesondere dentalen, Instruments (1), wobei an dem Instrumentenkopf (2) ein Werkzeug (4) zum Einwirken auf eine Behandlungsstelle vorgesehen ist und wobei die Beleuchtungsvorrichtung (3 - 3G) umfasst: Eine Aufnahme oder Öffnung (5), in welcher das Werkzeug (4) aufnehmbar ist, einen die Aufnahme oder Öffnung (5) umgebenden Körper (6), welcher ein Strahlungsabgabeende (9) mit zumindest einer optisch leitenden, insbesondere transparenten, Strahlungsabgabefläche (8), ein Instrumentenanschlussende (16) und eine sich zwischen dem Strahlungsabgabeende (9) und dem Instrumentenanschlussende (16) erstreckende Mantelfläche (12) aufweist, zumindest ein am Körper (6) vorgesehenes Halbleiterelement (7), das zur Emission von elektromagnetischer Strahlung ausgebildet ist, so dass elektromagnetische Strahlung von der zumindest einen optisch leitenden Strahlungsabgabefläche (8) des Strahlungsabgabeendes (9) abgebbar ist und zumindest eine am Strahlungsabgabeende (9) des Körpers (6) der Beleuchtungsvorrichtung (3 - 3G) vorgesehene Öffnung (11) zur Abgabe von Fluid, so dass vom Strahlungsabgabeende (9) des Körpers (6) zusätzlich zumindest ein Fluid oder ein Fluidgemisch abgebbar ist, und und eine Aufnahme (36), zum Beispiel einen Rücksprung, an dem Instrumentenkopf (2), in der die Beleuchtungsvorrichtung (3 - 3G), insbesondere lösbar, aufnehmbar ist, wobei die Aufnahme (36) ausgebildet ist, Fluid an die Beleuchtungsvorrichtung (3 - 3G) zu übertragen und zumindest ein Teil der Aufnahme (36) als Fluidkanal dient, welcher der Beleuchtungsvorrichtung (3 - 3G) ein Fluid zuführt.

## Claims

1. A lighting device (3 - 3G) for an instrument head (2) of a medical, in particular dental, instrument (1), wherein a tool (4) for acting on a treatment site is provided on the instrument head (2), and wherein the lighting device (3 - 3G) comprises: a receptacle or an opening (5), in which the tool (4) can be accommodated, a body (6) surrounding the receptacle or opening (5) which comprises a radiation-emitting end (9) having at least one optically-conducting, in particular transparent radiation-emitting surface (8), an instrument-connecting end (16) and a lateral surface (12) extending between the radiation-emitting end (9) and the instrument-connecting end (16), at least one semiconductor element (7) provided on the body (6) and designed for emission of electromagnetic radiation, so that electromagnetic radiation can be emitted from the at least one optically-conducting radiation-emitting surface (8) of the radiation-emitting end (9), and at least one opening (11) for dispensing fluid which is provided on the radiation-emitting end (9) of the body (6) of the lighting device (3 - 3G), so that in addition a fluid or fluid mixture can be dispensed from the radiation-emitting end (9) of the body (6) and a ring-shaped channel (40) which is provided on the lateral surface (12) for receiving a fluid, in particular water, and which is connected to the fluid dispensing opening (11).

2. The lighting device (3 - 3G) according to Claim 1, **characterized by**
at least one fluid channel (10, 10') which is provided in the lighting device (3-3G), in particular within the self-contained exterior circumference of the lateral surface (12) of the body (6), and which connects the at least one opening (11) for dispensing fluid provided on the radiation-emitting end (9) of the lighting device (3-3G) to the ring-shaped channel (40).

3. The lighting device (3 - 3G) according to Claim 1 or 2, **characterized by**
a plurality of fluid channels (10, 10') which are provided in the lighting device (3 - 3G)), in particular within the self-contained exterior circumference of the lateral surface (12) of the body (6), and which open into a shared fluid mixing chamber (13) in the lighting device (3 - 3G), wherein the fluid mixing chamber (13) is connected to the at least one opening (11) for dispensing fluid.

4. The lighting device (3 - 3G) according to any one of Claims 1 - 3, **characterized by**
a plurality of fluid channels (10, 10', 40) which are provided in the lighting device (3 - 3G), in particular within the self-contained exterior circumference of the lateral surface (12) of the body (6), wherein at least one fluid channel (10') comprises an opening (14) for connection to a fluid source on the lateral surface (12) of the body (6) of the lighting device (3 - 3G) and at least one fluid channel (10) comprises an opening (15) for connection to a fluid source on the instrument-connecting end (16) of the body (6) of the lighting device (3 - 3G).

5. The lighting device (3 - 3G) according to any one of Claims 1 - 4, **characterized by**
a plurality of optically-conducting radiation-emitting surfaces (8) and a plurality of openings (11) for dispensing fluid, wherein at least one radiation-emitting surface (8) is arranged between two openings (11) for dispensing fluid.

6. The lighting device (3 - 3G) according to any one of Claims 1 - 5, **characterized in that**
a plurality of electrically series-connected semiconductor elements (7) which are connected by means of an electric conductor (17) are provided, wherein the electric conductor (17) is arranged at a distance from the at least one fluid channel (10, 10', 40) provided in the lighting device (3 - 3G).

7. The lighting device (3 - 3G) according to any one of Claims 1 - 6, **characterized in that**
the lighting device (3 - 3G) is constructed of multiple layers (18, 19), in particular multiple layers (18, 19) of different materials.

8. The lighting device (3 - 3G) according to Claim 7, **characterized in that**
at least one of the layers comprises metallic material (19, 19A, 19B, 19C) and/or plastic and/or glass (20), in particular the layer that forms the radiation-emitting end (9) of the lighting device (3 - 3G).

9. The lighting device (3 - 3G) according to Claim 7 or 8, **characterized in that**
the lighting device (3 - 3G) comprises two layers (18B, 19C), wherein a first layer (19C) in which the ring-shaped channel (40) is provided is made of metal, wherein the first layer (19C) comprises a flange (41) and/or a shoulder (42) for connecting or supporting the second layer (18B) and wherein at least one optical radiation conductor (21) is accommodated in at least one bore of the first layer (19C).

10. The lighting device (3 - 3G) according to any one of Claims 1 - 9, **characterized in that**
a plurality of semiconductor elements (7) is provided, wherein each semiconductor element (7) is assigned its own optical radiation conductor (21) which is arranged so that it conducts the electromagnetic radiation emitted by the semiconductor elements (7) in the direction of the radiation-emitting end (9) of the lighting device (3 - 3G).

11. The lighting device (3 - 3G) according to any one of Claims 1 - 8, **characterized in that**
the radiation-emitting end (9) of the lighting device (3 - 3G) only comprises a single transparent light- or radiation-emitting surface (8), which is transparent for visible light in particular, and which is designed substantially in a ring shape or extends around the opening (5) in which the tool (4) can be accommodated.

12. An instrument head (2) of a medical, in particular dental, instrument (1), **characterized by** a lighting device (3 - 3G) according to any one of Claims 1 - 11.

13. The instrument head (2) of a medical, in particular dental, instrument (1), according to Claim 12, **characterized by**
a receptacle (36), for example a recess, on the instrument head (2), in which the lighting device (3 - 3G) can be received, in particular detachably received, wherein preferably the receptacle (36) is designed to convey fluid to the lighting device (3 - 3G).

14. The instrument head (2) of a medical, in particular dental, instrument (1), according to Claim 13, **characterized in that**
at least a portion of the receptacle (36) serves as a fluid channel which supplies a fluid to the lighting device (3 - 3G) or that at least a portion of the receptacle (36) together with the lighting device (3 - 3G) for example, a recess of the lighting device (3 - 3G), forms at least a section of the fluid channel of the lighting device (3 - 3G), or that the recess (36) is designed at least as a portion of a channel for example, ring-shaped or as a ring channel, which delivers a fluid to a plurality of fluid channels (10, 10').

15. An instrument head (2) of a medical, in particular dental, instrument (1), **characterized by**
a lighting device (3 - 3G) for an instrument head (2) of a medical, in particular dental, instrument (1), wherein a tool (4) for acting on a treatment site is provided on the instrument head (2), and wherein the lighting device (3 - 3G) comprises: a receptacle or an opening (5), in which the tool (4) can be accommodated, a body (6) surrounding the receptacle or opening (5) which comprises a radiation-emitting end (9) having at least one optically-conducting, in particular transparent radiation-emitting surface (8), an instrument-connecting end (16) and a lateral surface (12) extending between the radiation-emitting end (9) and the instrument-connecting end (16), at least one semiconductor element (7) provided on the body (6) and designed for emission of electromagnetic radiation, so that electromagnetic radiation can be emitted from the at least one optically-conducting radiation-emitting surface (8) of the radiation-emitting end (9), and at least one opening (11) for dispensing fluid which is provided on the radiation-emitting end (9) of the body (6) of the lighting device (3 - 3G), so that in addition a fluid or fluid mixture can be dispensed from the radiation-emitting end (9) of the body (6) and a receptacle (36), for example a recess, on the instrument head (2), in which the lighting device (3 - 3G) can be received, in particular detachably received, wherein the receptacle (36) is designed to convey fluid to the lighting device (3 - 3G) and at least a portion of the receptacle (36) serves as a fluid channel which supplies a fluid to the lighting device (3 - 3G).

## Revendications

1. Dispositif d'éclairage (3 - 3G) pour une tête d'instrument (2) d'un instrument (1) médical, en particulier dentaire, dans lequel on prévoit, au niveau de la tête d'instrument (2), un outil (4) pour agir sur une zone de traitement et dans lequel le dispositif d'éclairage (3 - 3G) comprend: un logement ou une ouverture (5) dans lequel/laquelle l'outil (4) peut être logé, un corps (6) entourant le logement ou l'ouverture (5), lequel présente une extrémité d'émission de rayonnement (9) avec au moins une surface d'émission de rayonnement (8) à conductivité optique, en particulier transparente, une extrémité de raccordement d'instrument (16) et une surface d'enveloppe (12) s'étendant entre l'extrémité d'émission de rayonnement (9) et l'extrémité de raccordement d'instrument (16), au moins un élément semi-conducteur (7) prévu sur le corps (6), lequel est réalisé pour l'émission d'un rayonnement électromagnétique de manière à ce qu'un rayonnement électromagnétique puisse être émis par l'au moins une surface d'émission de rayonnement (8) à conductivité optique de l'extrémité d'émission de rayonnement (9) et au moins une ouverture (11) prévue à l'extrémité d'émission de rayonnement (9) du corps (6) du dispositif d'éclairage (3 - 3G) pour l'émission de fluide de manière à ce qu'au moins un fluide ou mélange de fluides puisse être émis additionnellement par l'extrémité d'émission de rayonnement (9) du corps (6), et un canal annulaire (40) prévu sur la surface d'enveloppe (12) pour la réception d'un fluide, en particulier de l'eau, lequel est relié à l'ouverture d'émission de fluide (11).

2. Dispositif d'éclairage (3 - 3G) selon la revendication 1, **caractérisé par**
au moins un canal de fluide (10, 10') prévu dans le dispositif d'éclairage (3 - 3G), en particulier à l'intérieur de la périphérie extérieure refermée sur elle-même de la surface d'enveloppe (12) du corps (6), lequel relie l'au moins une ouverture (11) pour l'émission de fluide prévue à l'extrémité d'émission de rayonnement (9) du dispositif d'éclairage (3 - 3G) au canal annulaire (40).

3. Dispositif d'éclairage (3 - 3G) selon la revendication 1 ou 2, **caractérisé par**
plusieurs canaux de fluide (10, 10') prévus dans le dispositif d'éclairage (3 - 3G), en particulier à l'intérieur de la périphérie extérieure refermée sur elle-même de la surface d'enveloppe (12) du corps (6), lesquels débouchent dans une chambre (13) de mélange de fluides commune dans le dispositif d'éclairage (3 - 3G), dans lequel la chambre (13) de mélange de fluides est reliée à l'au moins une ouverture (11) pour l'émission de fluide.

4. Dispositif d'éclairage (3 - 3G) selon l'une des revendications 1 - 3, **caractérisé par**
plusieurs canaux de fluide (10, 10', 40) prévus dans le dispositif d'éclairage (3 - 3G), en particulier à l'intérieur de la périphérie extérieure refermée sur elle-même de la surface d'enveloppe (12) du corps (6), dans lequel au moins un canal de fluide (10') présente une ouverture (14) pour la liaison avec une source de fluide au niveau de la surface d'enveloppe (12) du corps (6) du dispositif d'éclairage (3 - 3G) et au moins un canal de fluide (10) présentant une ouverture (15) pour la liaison avec une source de fluide à l'extrémité de raccordement d'instrument (16) du corps (6) du dispositif d'éclairage (3 - 3G).

5. Dispositif d'éclairage (3 - 3G) selon l'une des revendications 1 - 4, **caractérisé par**
plusieurs surfaces d'émission de rayonnement (8) à conductivité optique et plusieurs ouvertures (11) pour l'émission de fluide, dans lequel, entre deux ouvertures (11) pour l'émission de fluide, au moins une surface d'émission de rayonnement (8) est disposée.

6. Dispositif d'éclairage (3 - 3G) selon l'une des revendications 1 - 5, **caractérisé en ce que**
l'on prévoit plusieurs éléments semi-conducteurs (7) connectés électriquement en série, reliés au moyen d'un conducteur électrique (17), dans lequel le conducteur électrique (17) est disposé à distance de l'au moins un canal de fluide (10, 10', 40) prévu dans le dispositif d'éclairage (3 - 3G).

7. Dispositif d'éclairage (3 - 3G) selon l'une des revendications 1 - 6, **caractérisé en ce que**
le dispositif d'éclairage (3 - 3G) est construit à partir de plusieurs couches (18, 19), en particulier à partir de plusieurs couches de matériaux différents.

8. Dispositif d'éclairage (3 - 3G) selon la revendication 7, **caractérisé en ce que**
au moins l'une des couches comprend un matériau métallique (19, 19A, 19B, 19C) et/ou du plastique et/ou du verre (20), en particulier celle des couches qui forme l'extrémité d'émission de rayonnement (9) du dispositif d'éclairage (3 - 3G).

9. Dispositif d'éclairage (3 - 3G) selon la revendication 7 ou 8, **caractérisé en ce que**
le dispositif d'éclairage (3 - 3G) comprend deux couches (18B, 19C), dans lequel une première couche (19C) dans laquelle le canal annulaire (40) est prévu se compose de métal, dans lequel la première couche (19C) présente une bride (41) et/ou un épaulement (42) pour la liaison ou le positionnement de la deuxième couche (18B) et dans lequel, dans au moins un alésage de la première couche (19C), au moins un conducteur de rayonnement optique (21) est logé.

10. Dispositif d'éclairage (3 - 3G) selon l'une des revendications 1 - 9, **caractérisé en ce que**
plusieurs éléments semi-conducteurs (7) sont prévus, dans lequel on associe à chaque élément semi-conducteur (7) un conducteur de rayonnement optique (21) propre qui est disposé d'une manière telle, qu'il conduit le rayonnement électromagnétique émis par les éléments semi-conducteurs (7) en direction de l'extrémité d'émission de rayonnement (9) du dispositif d'éclairage (3 - 3G).

11. Dispositif d'éclairage (3 - 3G) selon l'une des revendications 1 - 8, **caractérisé en ce que**
l'extrémité d'émission de rayonnement (9) du dispositif d'éclairage (3 - 3G) présente seulement une surface d'émission de rayonnement ou de lumière (8) transparente, en particulier pour la lumière visible, laquelle est réalisée sensiblement en forme d'anneau ou bien s'étend autour de l'ouverture (5) dans laquelle l'outil (4) peut être logé.

12. Tête d'instrument (2) d'un instrument (1) médical, en particulier dentaire, **caractérisée par**
un dispositif d'éclairage (3 - 3G) selon l'une des revendications 1 - 11.

13. Tête d'instrument (2) d'un instrument (1) médical, en particulier dentaire selon la revendication 12, **caractérisée par**
un logement (36), par exemple un emboitement, au niveau de la tête d'instrument (2), dans lequel le dispositif d'éclairage (3 - 3G) peut être logé, en particulier de façon détachable, dans lequel le logement (36) est de préférence réalisé pour transmettre du fluide au dispositif d'éclairage (3 - 3G).

14. Tête d'instrument (2) d'un instrument (1) médical, en particulier dentaire selon la revendication 13, **caractérisée en ce que**
au moins une partie du logement (36) sert de canal de fluide qui amène un fluide au dispositif d'éclairage (3 - 3G) ou bien **en ce qu'**au moins une partie du logement (36) forme, ensemble avec le dispositif d'éclairage (3 - 3G), par exemple d'un évidement du dispositif d'éclairage (3 - 3G), au moins un tronçon du canal de fluide du dispositif d'éclairage (3 - 3G), ou bien **en ce que** le logement (36) est réalisé au moins en tant que partie d'un canal, par exemple en forme d'anneau ou en tant que canal annulaire qui émet un fluide à l'attention de plusieurs canaux de fluide (10, 10').

15. Tête d'instrument (2) d'un instrument (1) médical, en particulier dentaire, **caractérisée par** un dispositif d'éclairage (3 - 3G) pour une tête d'instrument (2) d'un instrument (1) médical, en particulier dentaire, dans lequel on prévoit, au niveau de la tête d'instrument (2), un outil (4) pour agir sur une zone de traitement et dans lequel le dispositif d'éclairage (3 - 3G) comprend: un logement ou une ouverture (5) dans lequel/laquelle l'outil (4) peut être logé, un corps (6) entourant le logement ou l'ouverture (5), lequel présente une extrémité d'émission de rayonnement (9) avec au moins une surface d'émission de rayonnement (8) à conductivité optique, en particulier transparente, une extrémité de raccordement d'instrument (16) et une surface d'enveloppe (12) s'étendant entre l'extrémité d'émission de rayonnement (9) et l'extrémité de raccordement d'instrument (16), au moins un élément semi-conducteur (7) prévu sur le corps (6), lequel est réalisé pour l'émission d'un rayonnement électromagnétique de manière à ce qu'un rayonnement électromagnétique puisse être émis par l'au moins une surface d'émission de rayonnement (8) à conductivité optique de l'extrémité d'émission de rayonnement (9) et au moins une ouverture (11) prévue à l'extrémité d'émission de rayonnement (9) du corps (6) du dispositif d'éclairage (3 - 3G) pour l'émission de fluide, de manière à ce qu'au moins un fluide ou un mélange de fluides puisse être émis additionnellement par l'extrémité d'émission de rayonnement (9) du corps (6), et un logement (36), par exemple un emboitement au niveau de la tête d'instrument (2) dans lequel le dispositif d'éclairage (3, 3G) peut être logé, en particulier de façon détachable, dans lequel le logement (36) est réalisé pour transmettre du fluide au dispositif d'éclairage (3 - 3G) et au moins une partie du logement (36) servant de canal de fluide, lequel amène un fluide au dispositif d'éclairage (3 - 3G).
